# EUROPEAN PATENT APPLICATION

(11) **EP 4 606 897 A1**
(43) Date of publication of application: **27.08.2025**
(21) Application number: 23878387.2
(22) Date of filing: 07.09.2023
(51) Int. Cl.: C12N 15/62, A61K 35/17, A61K 48/00, A61P 35/00, A61P 37/02, A61P 43/00, C07K 14/705, C07K 16/28, C07K 19/00, C12N 5/10, C12N 15/13, C12N 15/63

(54) **NUCLEIC ACID MOLECULE ENCODING CAR AND VECTOR CONTAINING SAME, CAR, IMMUNE CELL CONTAINING CAR AND PHARMACEUTICAL COMPOSITION CONTAINING SAID CELL, AND METHOD FOR PRODUCING IMMUNE CELL CONTAINING CAR**

(30) Priority: 20.10.2022 JP 2022168686
(71) Applicant: Mie University, Tsu-shi, Mie 514-8507 (JP); SYSMEX CORPORATION, Kobe-shi Hyogo 651-0073 (JP)
(72) Inventor: SHIKU, Hiroshi, Tsu-shi, Mie 514-8507 (JP); AKAHORI, Yasushi, Tsu-shi, Mie 514-8507 (JP); MAETA, Shingo, Kobe-shi, Hyogo 651-0073 (JP); FUKUNAGA, Atsushi, Kobe-shi, Hyogo 651-0073 (JP)
(74) Representative: Hoffmann Eitle
(86) International application number: PCT/JP2023/032627
(87) International publication number: WO 2024/084852

(57) **Abstract**

The present invention relates to a nucleic acid molecule having a nucleotide sequence encoding a chimeric antigen receptor (CAR). The present invention relates to a vector including the nucleic acid molecule. The present invention relates to a CAR. The present invention relates to an immune cell including the CAR. The present invention relates to a pharmaceutical composition including the immune cell including the CAR. The present invention relates to a method for producing an immune cell including the CAR.

## Description

### FIELD OF THE INVENTION

The present invention relates to a nucleic acid molecule having a nucleotide sequence encoding a chimeric antigen receptor (hereinafter, also referred to as "CAR"). The present invention relates to a vector including the nucleic acid molecule. The present invention relates to a CAR. The present invention relates to an immune cell including the CAR. The present invention relates to a pharmaceutical composition for treating a malignant tumor including the immune cell including the CAR. The present invention relates to a method for producing an immune cell including the CAR.

### BACKGROUND

The CAR is a receptor protein produced by genetically engineering fusion of an extracellular domain including an antigen binding region, a transmembrane domain, and an intracellular domain that transmits an activation signal of an immune cell. For example, as described in US Patent No. 7,741,465, a single-chain antibody that recognizes an antigen expressed in a tumor cell is used for the antigen binding region of the CAR. In recent years, cancer immunotherapy has attracted attention in which a gene encoding a CAR is introduced into an immune cell, and an immune cell expressing the CAR on the cell surface is transplanted to a patient to treat cancer. When the immune cell expressing the CAR recognizes an antigen of a tumor cell in vivo, the immune cell is activated to express cytotoxic molecules such as Fas ligand, perforin, and granzyme and cytokines, and exerts an antitumor effect. On the other hand, it is known that production of cytokines by immune cells expressing a CAR may cause a cytokine release syndrome (CRS).

### PRIOR ART

### PATENT DOCUMENT

[Patent document 1] U.S. Patent No. 7,741,465

### SUMMARY OF THE INVENTION

### PROBLEM TO BE SOLVED BY THE INVENTION

An object of the present invention is to provide a CAR capable of suppressing production of a cytokine by the immune cell when expressed in the immune cell. An object of the present invention is to provide a nucleic acid molecule encoding such a CAR and a vector including the nucleic acid molecule. An object of the present invention is to provide an immune cell including such a CAR, a pharmaceutical composition including the immune cell, and a method for producing the immune cell.

### MEANS FOR SOLVING THE PROBLEM

The present inventors have found that when a predetermined amino acid residue in framework region 3 (FR3) of the light chain variable region of the antigen binding region of a CAR is a basic amino acid residue, production of a cytokine by the immune cell including the CAR can be suppressed, thereby completing the present invention. Therefore, the invention described in the following items [1] to [21] is provided.
[1] A nucleic acid molecule comprising a nucleotide sequence encoding a chimeric antigen receptor, wherein
   the nucleic acid molecule comprises a segment encoding an extracellular domain, a segment encoding a transmembrane domain, and a segment encoding an intracellular domain,
   the segment encoding the extracellular domain comprises a nucleotide sequence encoding an antigen binding region comprising a light chain variable region and a heavy chain variable region, and
   at least three codons in the nucleotide sequence encoding framework region 3 of the light chain variable region defined by the Kabat method are codons encoding basic amino acid residues.
[2] The nucleic acid molecule according to item 1, wherein said at least three codons comprise at least three codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, a codon encoding an amino acid residue at position 76, a codon encoding an amino acid residue at position 77, a codon encoding an amino acid residue at position 79, and a codon encoding an amino acid residue at position 81 of the light chain variable region.
[3] The nucleic acid molecule according to item 1, wherein 3 or more and 5 or less codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, a codon encoding an amino acid residue at position 76, a codon encoding an amino acid residue at position 77, a codon encoding an amino acid residue at position 79, and a codon encoding an amino acid residue at position 81 of the light chain variable region amino acid residue at position 60amino acid residue at position 63amino acid residue at position 65amino acid residue at position 67amino acid residue at position 70tamino acid residue at position 72amino acid residue at position 74amino acid residue at position 76amino acid residue at position 77amino acid residue at position 79amino acid residue at position 81 defined by the Kabat method are codons encoding basic amino acid residues.
[4] The nucleic acid molecule according to item 1, wherein the antigen binding region comprises a single-chain antibody, and the single-chain antibody is a single-chain antibody that binds to: a complex of a MAGE-A4 derived peptide and HLA-A2, a complex of a PRAME derived peptide and HLA-A24, CD19, BCMA, or CEA.
[5] The nucleic acid molecule according to item 1, wherein the transmembrane domain comprises a transmembrane region of a protein selected from the group consisting of an α chain of a T cell receptor, a β chain of a T cell receptor, CD3ε, CD3ζ, CD4, CD5, CD8, CD9, CD16, CD22, CD28, CD33, CD37, CD45, CD64, CD80, CD86, CD134, CD154, 4-1BB, ICOS, and GITR.
[6] The nucleic acid molecule according to item 1, wherein the intracellular domain comprises a signaling domain of at least one protein selected from the group consisting of CD3ζ, CD3γ, CD3δ, CD3ε, CD5, CD22, CD66d, CD79a, CD79b, FcRγ, and FcRβ.
[7] The nucleic acid molecule according to item 6, wherein the segment encoding the intracellular domain further comprises a nucleotide sequence encoding a co-stimulatory domain, and
   the co-stimulatory domain is a co-stimulatory domain of at least one protein selected from the group consisting of 4-1B B,CD28,GITR,CD2,CD5,CD8,CD9,CD16,CD22,CD33,CD37,CD64,CD80,CD86,CD 134, CD154, and ICOS.
[8] The nucleic acid molecule according to item 1, further comprising a segment encoding a hinge domain between the nucleotide sequence encoding the antigen binding region and the segment encoding the transmembrane domain.
[9] The nucleic acid molecule according to item 1, wherein the nucleic acid molecule is DNA or RNA.
[10] A vector comprising the nucleic acid molecule according to any one of items 1 to 9.
[11] A chimeric antigen receptor comprising an extracellular domain, a transmembrane domain, and an intracellular domain, wherein
   the extracellular domain comprises an antigen binding region comprising a light chain variable region and a heavy chain variable region, and
   at least three amino acid residues in framework region 3 of the light chain variable region defined by the Kabat method are basic amino acid residues.
[12] The chimeric antigen receptor according to item 11, wherein said at least three amino acid residues comprise at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, an amino acid residue at position 76, an amino acid residue at position 77, an amino acid residue at position 79, and an amino acid residue at position 81 in the light chain variable region.
[13] The chimeric antigen receptor according to item 11, wherein 3 or more and 5 or less amino acid residues selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, an amino acid residue at position 76, an amino acid residue at position 77, an amino acid residue at position 79, and an amino acid residue at position 81 in the light chain variable region defined by the Kabat method are basic amino acid residues.
[14] The chimeric antigen receptor according to item 11, wherein the antigen binding region comprises a single-chain antibody, and the single-chain antibody is a single-chain antibody that binds to a complex of a MAGE-A4 derived peptide and HLA-A2, a complex of a PRAME derived peptide and HLA-A24, CD19, BCMA, or CEA.
[15] The chimeric antigen receptor according to item 11, wherein the transmembrane domain comprises a transmembrane region of any one protein selected from the group consisting of an α chain of a T cell receptor, a β chain of a T cell receptor, CD3ε, CD3ζ, CD4,CD5,CD8,CD9,CD16,CD22,CD28,CD33,CD37,CD45,CD64,CD80,CD86,CD 134, CD154,4-1BB,ICOS, and GITR.
[16] The chimeric antigen receptor according to item 11, wherein the intracellular domain comprises a signaling domain of at least one protein selected from the group consisting of CD3ζ, CD3γ, CD3δ, CD3ε, CD5,CD22,CD66d,CD79a,CD79b,FcRγ, and FcRβ.
[17] The chimeric antigen receptor according to item 16, wherein the intracellular domain further comprises a co-stimulatory domain, and
   the co-stimulatory domain is a co-stimulatory domain of at least one protein selected from the group consisting of 4-1BB, CD28,GITR,CD2,CD5,CD8,CD9,CD16,CD22,CD33,CD37,CD64,CD80,CD86,CD 134, CD154, and ICOS.
[18] The chimeric antigen receptor according to item 11, further comprising a hinge domain between the antigen binding region and the transmembrane domain.
[19] An immune cell comprising the chimeric antigen receptor according to any one of items 11 to 18.
[20] A pharmaceutical composition for treating a malignant tumor comprising the immune cell according to item 19.
[21] A method for producing an immune cell comprising a chimeric antigen receptor, comprising introducing the nucleic acid molecule according to any one of items 1 to 9 into an immune cell, and expressing the chimeric antigen receptor in the immune cell.
[22] A method for producing an immune cell comprising a chimeric antigen receptor, comprising introducing the vector according to item 10 into an immune cell, and expressing the chimeric antigen receptor in the immune cell.

### EFFECT OF THE INVENTION

According to the present invention, an immune cell including the CAR in which the production of the cytokine is suppressed can be obtained.

### BRIEF DESCRIPTION OF THE DRAWINGS

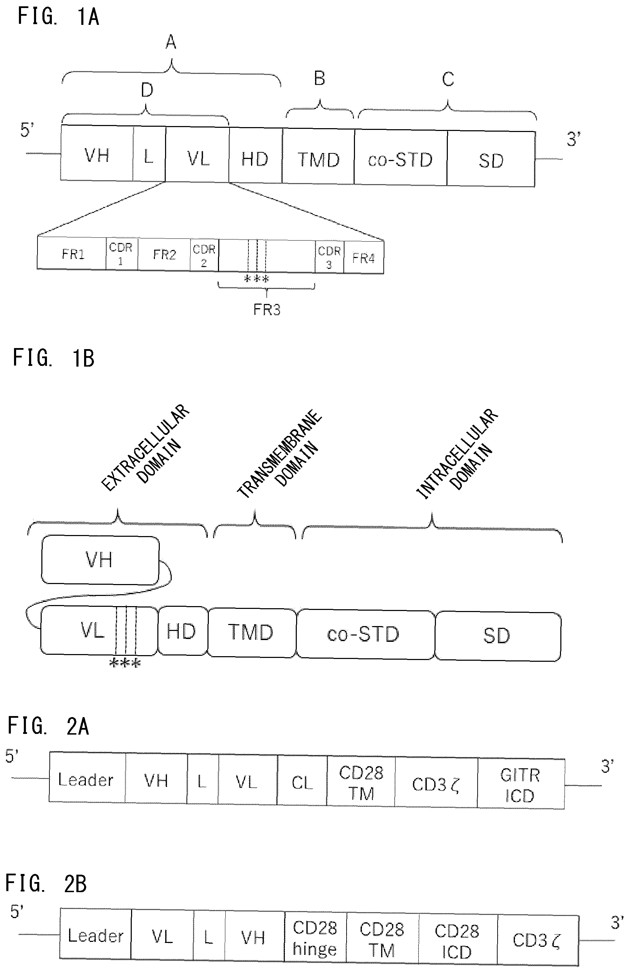

[Fig.1A] is a diagram schematically showing an example of a nucleic acid molecule of the present embodiment. Also shown is an enlarged diagram of the segment encoding the light chain variable region (VL) in a nucleic acid molecule. In the figure, A denotes a segment encoding the extracellular domain, B denotes a segment encoding the transmembrane domain, and C denotes a segment encoding the intracellular domain. D denotes a segment encoding an antigen binding region included in the extracellular domain. In these segments, VH denotes a segment encoding a heavy chain variable region, L denotes a segment encoding a linker, HD denotes a segment encoding a hinge domain, TMD denotes a segment encoding a transmembrane domain, co-STD denotes a segment encoding a co-stimulatory domain, and SD denotes a segment encoding a signaling domain. In the enlarged diagram of the VL, dashed lines marked with * indicate codons encoding basic amino acid residues. In Fig. 1A, in the segment encoding FR3, there are three codons encoding basic amino acid residues, but the present invention is not limited thereto.
[Fig. 1B] is a diagram schematically showing an example of a CAR of the present embodiment. In the figure, VH denotes a heavy chain variable region, VL denotes a light chain variable region, HD denotes a hinge domain, TMD denotes a transmembrane domain, co-STD denotes a co-stimulatory domain, and SD denotes a signaling domain. The curve connecting VH and VL indicates the linker. In VL, the dashed line marked with * indicates basic amino acid residues. In Fig. 1B, there are three basic amino acid residues in FR3, but the present invention is not limited thereto.
[Fig. 2A] is a diagram schematically showing a nucleic acid molecule encoding the CAR of Examples 1 to 3.
[Fig. 2B] is a diagram schematically showing a nucleic acid molecule encoding the CAR of Example 4.
[Fig. 3] This is a graph in which PBS or CAR-T cells were administered to mice transplanted with tumor cells, and then the tumor area (average value) of each mouse was recorded.

### DETAILED DESCRIPTION OF THE PREFERRED EMBODIMENTS

### 1. Nucleic acid molecule

The nucleic acid molecule of the present embodiment has a nucleotide sequence encoding a CAR. In the present specification, the "nucleotide sequence" is synonymous with "base sequence" or "nucleic acid sequence". The nucleotide sequence refers to a one-dimensional arrangement (order) of nucleotides in a nucleic acid molecule. Therefore, a nucleic acid molecule encoding a polypeptide has a nucleotide sequence encoding the polypeptide. In the present specification, the expression "having a nucleotide sequence" means both composed of the nucleotide sequence and including the nucleotide sequence. As used herein, the term "polypeptide" includes a protein molecule, a part of region within a protein molecule, and a fragment of a protein molecule. In the present specification, a part of region in a nucleic acid molecule may be referred to as a "segment", and a part of region in a protein molecule may be referred to as a "domain".

As exemplified in Fig. 1A, the nucleic acid molecule of the present embodiment includes, in order from the 5' end, a segment encoding an extracellular domain, a segment encoding a transmembrane domain, and a segment encoding an intracellular domain. The segment encoding the extracellular domain includes a nucleotide sequence encoding an antigen binding region including a light chain variable region and a heavy chain variable region. In the present specification, the "antigen binding region including a light chain variable region and a heavy chain variable region" refers to a domain including at least one light chain variable region and at least one heavy chain variable region or capable of binding to a predetermined antigen via these regions. Examples of the antigen binding region include a single-chain antibody. The single-chain antibody is also referred to as an scFv, and the light chain variable region and the heavy chain variable region are linked via a peptide linker, and are a part of region of the CAR construct of the present embodiment. In the nucleic acid molecule of Figure 1A, the segment encoding the antigen binding region includes a nucleotide sequence encoding a single-chain antibody composed of VH, L and VL. Details of each segment of the nucleic acid molecule of the present embodiment and the CAR encoded by the nucleic acid molecule are described later.

As exemplified in Fig. 1A, the nucleic acid molecule of the present embodiment has a characteristic that at least three codons in the nucleotide sequence encoding FR3 (hereinafter, also referred to as "light chain FR3") of the light chain variable region are codons encoding basic amino acid residues. That is, the CAR encoded by the nucleic acid molecule of the present embodiment has an antigen binding region in which at least three amino acid residues of the light chain FR3 are basic amino acid residues. This antigen binding region is hereinafter also referred to as a "modified antigen binding region". A CAR having a modified antigen binding region is hereinafter also referred to as a "post-modification CAR". The nucleic acid molecule of the present embodiment can be said to be a nucleic acid molecule encoding the post-modification CAR. As used herein, the term "codon" refers to three consecutive nucleotides of DNA or RNA.

The nucleic acid molecule encoding the modified antigen binding region can be obtained by modification of a codon described later with respect to the nucleic acid molecule encoding the original antigen binding region. In the present specification, the "original antigen binding region" refers to an antigen binding region before being modified, in which the number of basic amino acid residues in the light chain FR3 is two or less. That is, in the nucleotide sequence encoding the light chain FR3 in the nucleotide sequence encoding the original antigen binding region, the number of codons encoding basic amino acid residues is two or less. Hereinafter, the CAR having the original antigen binding region is also referred to as a "pre-modification CAR".

The framework region (FR) is a region other than the complementarity determining region (CDR) present in each of the light chain variable region and the heavy chain variable region of an antibody. The FR serves as a scaffold linking the three CDRs and contributes to the structural stability of the CDRs. Therefore, the amino acid sequence of the FR is highly conserved among antibodies of the same species. In the variable region of each of the heavy chain and the light chain, there are three CDRs of CDR1, CDR2 and CDR3 and four FRs of FR1, FR2, FR3 and FR4. These are arranged in the order of FR1, CDR1, FR2, CDR2, FR3, CDR3 and FR4 from the N-terminus of the variable region. Hereinafter, the CDR of the heavy chain may be referred to as "HCDR", and the CDR of the light chain may be referred to as "LCDR".

In the art, the method of numbering the amino acid residue of a CDR (hereinafter, also referred to as "numbering method") to define the boundary and length of the CDR is known. When the amino acid residue of the CDR is numbered by the numbering method, the amino acid residue of the FR is also numbered. The number numbered to the amino acid residue by the numbering method indicates the position of the amino acid residue in the amino acid sequence of the light chain or the heavy chain. Examples of the numbering method include the Kabat method (Kabat EA. et al., Sequences of Proteins of Immunological Interest., NIH publication No.91 - 3242), the Chothia method (Chothia C. and Lesk AM., Canonical Structures for the Hypervariable Regions of Immunoglobulins., J Mol Biol., vol. 196, p.901 - 917, 1987), the IMGT method (Lefranc MP. et al., Developmental and Comparative Immunology 29 (2005) 185-203), the Honegger method (Honegger A. et al., Yet Another Numbering Scheme for Immunoglobulin Variable Domains: An Automatic Modeling and Analysis Tool., J Mol Biol., vol.309, p.657-670, 2001), the ABM method, and the Contact method.

With respect to the antigen binding region, the boundaries, and lengths of CDRs and FRs in each variable region of the light chain and the heavy chain may be defined by any numbering method. In the present specification, boundaries and lengths of CDR and FR are defined by the Kabat method. For example, when the antigen binding region includes or consists of a single-chain antibody, according to the Kabat method, the light chain FR1 of the single-chain antibody is defined as an region consisting of amino acid residues 1 to 23 of the light chain variable region. The light chain FR2 of a single-chain antibody is defined as a region consisting of amino acid residues 35 to 49 of the light chain variable region. The light chain FR3 of a single-chain antibody is defined as a region consisting of amino acid residues 57 to 88 of the light chain variable region. The light chain FR4 of a single-chain antibody is defined as a region consisting of amino acid residues 98 to 109 of the light chain variable region. In the present specification, when the position of an amino acid residue in the light chain variable region of the antigen binding region is described, unless otherwise specified, the position of the amino acid residue represents a position defined by the Kabat method.

The nucleic acid molecule of the present embodiment can be obtained by linking a nucleic acid molecule encoding the extracellular domain, a nucleic acid molecule encoding the transmembrane domain, and a nucleic acid molecule encoding the intracellular domain in order from the 5' end. These nucleic acid molecules can be linked by known genetic recombination techniques and other molecular biological techniques. The nucleic acid molecule encoding the extracellular domain may consist of a nucleic acid molecule encoding a modified antigen binding region. Preferably, the nucleic acid molecule encoding the extracellular domain is obtained by linking a nucleic acid molecule encoding a modified antigen binding region and a nucleic acid molecule encoding a hinge domain by the above-described technology.

In the nucleotide sequence encoding the light chain FR3 of the original antigen binding region, changing three or more codons encoding amino acid residues that is not basic amino acid residues into codons encoding basic amino acid residues is hereinafter also referred to as "modify a codon" or" modification of a codon". The nucleic acid molecule encoding the modified antigen binding region can be obtained by performing modification of a codon of the nucleic acid molecule encoding the original antigen binding region. The amino acid residue that is not a basic amino acid residue is a neutral amino acid residue and/or an acidic amino acid residue, and is preferably a neutral amino acid residue. The nucleotide sequence encoding the modified antigen binding region and the nucleotide sequence encoding the original antigen binding region are preferably the same except for a portion where a codon is modified.

By such a modification of a codon, at least three codons in the nucleotide sequence encoding the light chain FR3 of the original antigen binding region become codons encoding basic amino acid residues, and a nucleic acid molecule encoding a modified antigen binding region can be obtained. The modification of a codon can be performed by substituting or inserting a codon in a nucleic acid molecule encoding the original antigen binding region.

The basic amino acid residue is a lysine residue, an arginine residue, and a histidine residue. Among them, an arginine residue and a lysine residue are preferable. The neutral amino acid residue is an alanine residue, an asparagine residue, a cysteine residue, a glycine residue, a glutamine residue, an isoleucine residue, a leucine residue, a methionine residue, a phenylalanine residue, a proline residue, a serine residue, a threonine residue, a tryptophan residue, a tyrosine residue, and a valine residue. The acidic amino acid residue is an aspartic acid residue and a glutamic acid residue.

In the nucleotide sequence encoding the light chain FR3, at least three codons introduced by modification of a codon may be all codons encoding an arginine residue or all codons encoding a lysine residue. Alternatively, in the nucleotide sequence encoding the light chain FR3, at least three codons introduced by modification of a codon may be codons a part of which encodes an arginine residue and the rest of which encodes a lysine residue.

The nucleic acid molecule of the present embodiment may be DNA or RNA. In the nucleotide sequence encoding the light chain FR3, the type of codon introduced by modification of a codon is not particularly limited as long as it encodes a basic amino acid residue. When the nucleic acid molecule of the present embodiment is DNA, examples of the codon encoding a basic amino acid residue include AGA,AGG,CGA,CGC,CGG and CGT encoding an arginine residue, AAA and AAG encoding a lysine residue, and CAC and CAT encoding a histidine residue. When the nucleic acid molecule of the present embodiment is RNA, examples of the codon encoding a basic amino acid residue include AGA,AGG,CGA,CGC,CGG and CGU encoding an arginine residue, AAA and AAG encoding a lysine residue, and CAC and CAU encoding a histidine residue.

In the nucleic acid molecule of the present embodiment, the number of codons encoding basic amino acid residues in the nucleotide sequence encoding the light chain FR3 is, for example, 3, 4, 5, 6, 7, 8, 9, 10, 11, 12, 13, or 14. The number of codons encoding basic amino acid residues in the nucleotide sequence encoding the light chain FR3 is preferably 3 or more and 6 or less, and more preferably 3 or more and 5 or less.

In the light chain FR3 of the modified antigen binding region, it is preferable that at least three basic amino acid residues derived from the modification of a codon are at the positions of amino acid residues obtained by removing the vernier zone residue and the unexposed residue from the amino acid sequence of the light chain FR3. The "vernier zone residue" is an amino acid residue that contributes to the structural stability of CDR in the amino acid sequence of FR. The "unexposed residue" is an amino acid residue that is folded inside the molecule and is not exposed to the surface. It is expected that even if the unexposed residues are modified, the effect of the modification is small or absent. For example, the amino acid residues obtained by removing the vernier zone residues and the unexposed residues from the amino acid sequence of the light chain FR3 are amino acid residues of the positions 57, 58, 59, 60, 61, 62, 63, 65, 67, 70, 72, 74, 76, 77, 79, 80 and 81.

In the nucleotide sequence encoding the modified antigen binding region, at least three codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, a codon encoding an amino acid residue at position 76, a codon encoding an amino acid residue at position 77, a codon encoding an amino acid residue at position 79, and a codon encoding an amino acid residue at position 81 in the light chain variable region are codons encoding a basic amino acid residue. More preferably, three or more and six or less codons selected from the above group are codons encoding basic amino acid residues. Particularly preferably, three or more and five or less codons selected from the above group are codons encoding basic amino acid residues. For example, each codon according to any one of the following 1) to 21) is a codon encoding a basic amino acid residue.

1) A codon encoding the amino acid residue at position 60, a codon encoding the amino acid residue at position 63, and a codon encoding the amino acid residue at position 65 in the light chain variable region;
2) A codon encoding the amino acid residue at position 60, a codon encoding the amino acid residue at position 63, and a codon encoding the amino acid residue at position 76 in the light chain variable region;
3) A codon encoding the amino acid residue at position 60, a codon encoding the amino acid residue at position 74, and a codon encoding the amino acid residue at position 76 in the light chain variable region;
4) A codon encoding the amino acid residue at position 63, a codon encoding the amino acid residue at position 65, and a codon encoding the amino acid residue at position 67 in the light chain variable region;
5) A codon encoding the amino acid residue at position 63, a codon encoding the amino acid residue at position 65, and a codon encoding the amino acid residue at position 70 in the light chain variable region;
6) A codon encoding the amino acid residue at position 63, a codon encoding the amino acid residue at position 65, and a codon encoding the amino acid residue at position 72 in the light chain variable region;
7) A codon encoding the amino acid residue at position 63, a codon encoding the amino acid residue at position 67, and a codon encoding the amino acid residue at position 70 in the light chain variable region;
8) A codon encoding the amino acid residue at position 63, a codon encoding the amino acid residue at position 67, and a codon encoding the amino acid residue at position 72 in the light chain variable region;
9) A codon encoding the amino acid residue at position 63, a codon encoding the amino acid residue at position 70, and a codon encoding the amino acid residue at position 72 in the light chain variable region;
10) A codon encoding the amino acid residue at position 65, a codon encoding the amino acid residue at position 67, and a codon encoding the amino acid residue at position 70 in the light chain variable region;
11) A codon encoding the amino acid residue at position 65, a codon encoding the amino acid residue at position 67, and a codon encoding the amino acid residue at position 72 in the light chain variable region;
12) A codon encoding the amino acid residue at position 65, a codon encoding the amino acid residue at position 70, and a codon encoding the amino acid residue at position 72 in the light chain variable region;
13) A codon encoding the amino acid residue at position 67, a codon encoding the amino acid residue at position 70, and a codon encoding the amino acid residue at position 72 in the light chain variable region;
14) A codon encoding the amino acid residue at position 70, a codon encoding the amino acid residue at position 72, and a codon encoding the amino acid residue at position 74 in the light chain variable region;
15) A codon encoding the amino acid residue at position 77th, a codon encoding the amino acid residue at position 79, and a codon encoding the amino acid residue at position 81 in the light chain variable region;
16) A codon encoding the amino acid residue at position 63, a codon encoding the amino acid residue at position 65, a codon encoding the amino acid residue at position 67, and a codon encoding the amino acid residue at position 70 in the light chain variable region;
17) A codon encoding the amino acid residue at position 63, a codon encoding the amino acid residue at position 65, a codon encoding the amino acid residue at position 67, and a codon encoding the amino acid residue at position 72 in the light chain variable region;
18) A codon encoding the amino acid residue at position 63, a codon encoding the amino acid residue at position 65, a codon encoding the amino acid residue at position 70, and a codon encoding the amino acid residue at position 72 in the light chain variable region;
19) A codon encoding the amino acid residue at position 63, a codon encoding the amino acid residue at position 67, a codon encoding the amino acid residue at position 70, and a codon encoding the amino acid residue at position 72 in the light chain variable region;
20) A codon encoding the amino acid residue at position 65, a codon encoding the amino acid residue at position 67, a codon encoding the amino acid residue at position 70, and a codon encoding the amino acid residue at position 72 in the light chain variable region; and
21) A codon encoding the amino acid residue at position 63, a codon encoding the amino acid residue at position 65, a codon encoding the amino acid residue at position 67, a codon encoding the amino acid residue at position 70, and a codon encoding the amino acid residue at position 72 in the light chain variable region.

In the nucleotide sequence encoding the modified antigen binding region, codons encoding at least three amino acid residues selected from the group consisting of positions 63, 65, 67, 70, and 72 of the light chain variable region and codons encoding at least one amino acid residue selected from the group consisting of positions 60, 74, 76, 77, 79, and 81 of the light chain variable region are preferably codons encoding basic amino acid residues.

In the nucleotide sequence encoding the light chain FR3 of the modified antigen binding region, when three or more and six or less codons are codons encoding basic amino acid residues, the three or more and six or less codons preferably include codons encoding three, four, or five amino acid residues selected from the group consisting of positions 63, 65, 67, 70, and 72 of the light chain variable region and codons encoding one, two, or three amino acid residues selected from the group consisting of positions 60, 74, 76, 77, 79, and 81 of the light chain variable region.

In the nucleotide sequence encoding the light chain FR3 of the modified antigen binding region, when three or more and five or less codons are codons encoding basic amino acid residues, the three or more and five or less codons preferably include codons encoding three or four amino acid residues selected from the group consisting of positions 63, 65, 67, 70, and 72 of the light chain variable region and codons encoding one or two amino acid residues selected from the group consisting of positions 60, 74, 76, 77, 79, and 81 of the light chain variable region.

In the nucleotide sequence encoding the modified antigen binding region, codons encoding at least three amino acid residues selected from the group consisting of positions 60, 74, 67, 79, and 81 of the light chain variable region and codons encoding at least one amino acid residue selected from the group consisting of positions 63, 65, 67, 70, and 72 of the light chain variable region are preferably codons encoding basic amino acid residues.

In the nucleotide sequence encoding the light chain FR3 of the modified antigen binding region, when three or more and six or less codons are codons encoding basic amino acid residues, the three or more and six or less codons preferably include codons encoding three, four, or five amino acid residues selected from the group consisting of positions 60, 74, 76, 77, 79, and 81 of the light chain variable region, and codons encoding one, two, or three amino acid residues selected from the group consisting of positions 63, 65, 67, 70, and 72 of the light chain variable region.

In the nucleotide sequence encoding the light chain FR3 of the modified antigen binding region, when three or more and five or less codons are codons encoding basic amino acid residues, the three or more and five or less codons preferably include codons encoding three or four amino acid residues selected from the group consisting of positions 60, 74, 76, 77, 79, and 81 of the light chain variable region and codons encoding one or two amino acid residues selected from the group consisting of positions 63, 65, 67, 70, and 72 of the light chain variable region.

In the nucleotide sequence encoding the modified antigen binding region, when a codon other than the codons encoding the amino acid residues at positions 60, 63, 65, 67, 70, 72, 74, 76, 77, 79 and 81 of the light chain variable region is modified, the modified codon is preferably a codon encoding amino acid residues at positions selected from the group consisting of positions 57, 58, 59, 61, and 62.

Since the CDR is involved in the affinity of the antigen binding region for the antigen, it is preferable that the nucleotide sequence encoding the CDR of the antigen binding region is not changed in the nucleic acid molecule of the present embodiment. That is, the amino acid sequence of the CDR of the modified antigen binding region and the nucleotide sequence encoding the same are preferably the same as the amino acid sequence of the CDR of the original antigen binding region and the nucleotide sequence encoding the same.

Hereinafter, the immune cell including the pre-modification CAR is also referred to as a "pre-modification immune cell", and the immune cell including the post-modification CAR is also referred to as a "post-modification immune cell". When the amino acid sequence of the post-modification CAR is the same as the amino acid sequence of the pre-modification CAR except that at least three amino acid residues of the light chain FR3 are basic amino acid residues, the post-modification immune cell and the pre-modification immune cell can be compared for the production of a cytokine and the cytotoxicity. In the post-modification immune cell, production of a cytokine is suppressed as compared with the pre-modification immune cell. The type of cytokine is not particularly limited, and examples thereof include IFNγ, tumor necrosis factor (TNF)-α, and interleukin (IL)-6. In the present specification, the "cytokine production is suppressed" means that the amount of cytokine released by the post-modification immune cell is smaller than the amount released by the pre-modification immune cell at a predetermined ratio, and a predetermined period from the mixing is elapsed. For example, the amount of cytokine released by the post-modification immune cell is 93% or less, 92% or less, 91% or less, 90% or less, 80% or less, 70% or less, 60% or less, 50% or less, 40% or less, 30% or less, or 20% or less of the amount released by the pre-modification immune cell. The amount of cytokine released by the immune cell including the CAR can be examined, for example, by co-culturing the immune cell and the tumor cell and then measuring the cytokine in the culture supernatant by a known method such as enzyme-linked immunosorbent assay (ELISA). Specifically, the method of Example 1 described below is illustrated.

Preferably, in the post-modification immune cell, the cytotoxicity on a tumor cell is maintained as compared with the pre-modification immune cell, and more preferably, the cytotoxicity is improved. In the present specification, the "cytotoxicity is maintained" means that the cytotoxicity by the pre-modification immune cell and the post-modification immune cell is substantially equivalent at a time point when the tumor cell and the immune cell are mixed at a predetermined ratio and a predetermined period from the mixing is elapsed. In a preferred embodiment, the "cytotoxicity is maintained" so that the cytotoxicity of the post-modification immune cell is 95% or more and 105% or less of the cytotoxicity of the pre-modification immune cell. If the amount of cytokine is greatly reduced even if the cytotoxicity of the post-modification immune cell is lower than the cytotoxicity of the pre-modification immune cell, a better therapeutic effect can be observed. When such an immune cell is administered to a patient, when the dose of the immune cell is increased, the cytotoxicity on the tumor cell is improved, and the cytokine may still be at a low level. That is, as long as the cytokine can be maintained at a low level, the dose of the immune cell can be appropriately adjusted to maintain or improve the cytotoxicity. The "cytotoxicity" can be evaluated in vitro. For example, the cytotoxicity can be evaluated by an index such as the survival number or survival rate of a tumor cell when the immune cell including the CAR and the tumor cell are co-cultured. Specifically, the method of Example 1 described below is illustrated. When solid cancer is targeted, the evaluation can also be performed by transplanting the tumor cell into a non-human animal such as a mouse, then administering the immune cell including the CAR, and measuring the size of the tumor. Specifically, the method of Example 5 described below is illustrated. In the case of using a blood cancer as a subject, it is also possible to evaluate by transplanting the tumor cell into a non-human animal such as a mouse, then administering the immune cell including the CAR, and measuring the number of tumor cells in the blood with a microscope, a flow cytometer, or the like.

As described above, the nucleic acid molecule encoding the modified antigen binding region can be obtained from the nucleic acid molecule encoding the original antigen binding region by known genetic recombination techniques and other molecular biological techniques. First, a primer set for modifying a codon is produced based on the nucleotide sequence of the nucleic acid molecule encoding the original antigen binding region. For example, when the modification of a codon is substitution of a codon, a primer set designed so that at least three codons in the nucleotide sequence encoding the light chain FR3 are substituted with codons encoding basic amino acid residues is produced. Then, by a PCR method using this primer set, a nucleic acid molecule encoding the original antigen binding region is amplified as a template, whereby a nucleic acid molecule encoding an antigen binding region in which at least three amino acid residues of the light chain FR3 are substituted with basic amino acid residues can be obtained. Alternatively, when the modification of a codon is insertion of a codon, a primer set designed to insert a codon encoding a basic amino acid residue at at least three positions in the nucleotide sequence encoding the light chain FR3 is produced. Then, by a PCR method using this primer set, a nucleic acid molecule encoding the original antigen binding region is amplified as a template, whereby a nucleic acid molecule encoding the antigen binding region in which basic amino acid residues are inserted at at least three positions of the light chain FR3 can be obtained.

As described above, since the nucleic acid molecule encoding the modified antigen binding region is obtained based on the nucleic acid molecule encoding the original antigen binding region, it is preferable that the original antigen binding region has available a nucleic acid molecule including a nucleotide sequence encoding the original antigen binding region. For example, when an E. coli clone having a plasmid DNA encoding the original antigen binding region is available, a nucleic acid molecule including a nucleotide sequence encoding the original antigen binding region can be obtained by extracting the plasmid DNA from the E. coli clone. In order to produce the primer set, it is preferable that the nucleotide sequence encoding the original antigen binding region is known or can be confirmed. When the antigen binding region includes or consists of a single-chain antibody, the nucleotide sequence encoding the single-chain antibody can be examined from known databases such as, for example, PDB,GeneBank,abYsis,IMGT. When a nucleic acid molecule including a nucleotide sequence encoding the original antigen binding region is possessed, the nucleotide sequence encoding the original antigen binding region can be examined by sequencing the nucleic acid molecule.

The modified antigen binding region and the original antigen binding region preferably bind to at least an antigen expressed in a tumor cell. The antigen may be an antigen that is also expressed in a normal cell or an antigen that is specifically expressed in a tumor cell. The antigen includes not only a full-length antigen but also a fragment of the antigen and a complex of the fragment of the antigen and a MHC (major histocompatibility complex) protein. Examples of the fragment of the antigen include a part of the antigen presented by the antigen-presenting cell or the tumor cell itself, a synthetic peptide composed of a part of the amino acid sequence of the antigen, and the like. The MHC protein is called HLA (human leukocyte antigen) in humans, and includes HLA-A, HLA-B, HLA-C, HLA-DR, HLA-DQ, HAL-DP, and the like. For example, when the antigen is a protein expressed in a tumor cell, the modified antigen binding region and the original antigen binding region can bind to a complex of a fragment of the antigen and an MHC protein.

As the type of antigen recognized by the antigen binding region, an antigen presents on the surface of a tumor cell and an antigen presents in a tumor cell are preferable. Examples of such an antigen include CD19,CD20,CD30,CD44,CD 133, MAGE-A1,MAGE-A2,MAGE-A3,MAGE-A4,PRAME,NY-ESO-1,SSX2,GP 100, MART-1,HER2, carcinoembryonic antigen (CEA), MUC-1, CA 125, glypican 3 (GPC3), B cell maturation antigen (BCMA), prostate-specific membrane antigen (PSMA), ganglioside GM2, and the like. The antigen recognized by the antigen binding region may be a complex of a fragment of the antigen and an MHC protein. This complex is formed when an antigen is fragmented in a tumor cell and the fragment is bound to MHC of the tumor cell. Then, the formed complex is presented on the surface of the tumor cell. Examples of the complex include a complex of a MAGE-A4 derived peptide and HLA-A2 (hereinafter also referred to as "MAGE-A4/HLA-A2 complex"), and a complex of a PRAME derived peptide and HLA-A24 (hereinafter also referred to as "PRAME/HLA-A24 complex"). Examples of the HLA-A2 forming a complex with the MAGE-A4 derived peptide include HLA-A*02:01, HLA-A*02:02, HLA-A*02:03, HLA-A*02:05, HLA-A*02:06, HLA-A*02:07, HLA-A*02:11, and the like. Among them, HLA-A*02:01 is preferable. Examples of the HLA-A24 forming a complex with a PRAME derived peptide include HLA-A*24:02 and HLA-A*24:03. Among them, HLA-A*24:02 is preferable. The MAGE-A4 derived peptide and the PRAME derived peptide may form a complex with HLA different from the above depending on the sequence of the peptide to be fragmented. The peptide derived from MAGE-A4 derived peptide and the PRAME derived peptide are oligopeptides consisting of a part (for example, 8 to 20 amino acids) of amino acid sequence of MAGE-A4 and PRAME, respectively. Examples of the MAGE-A4 derived peptide include oligopeptides composed of the amino acid sequence of GVYDGREHTV (SEQ ID NO: 1). Examples of the PRAME derived peptide include oligopeptides composed of an amino acid sequence of LYVDSLFFL (SEQ ID NO: 2).

The nucleic acid molecule itself encoding the original antigen binding region can be obtained by known genetic recombination techniques and other molecular biological techniques. When the antigen binding region includes or consists of a single-chain antibody, for example, a nucleic acid molecule encoding a single-chain antibody that binds to an antigen of a tumor cell can be isolated by phage display using an antibody phage library. Alternatively, a hybridoma that produces an antibody that binds to an antigen of a tumor cell can be produced, and a nucleic acid molecule encoding a single-chain antibody can be produced by a reverse transcription reaction and a PCR method using RNA extracted from the hybridoma. The hybridoma can be produced by a known method such as the method described in Kohler G. and Milstein C., Nature, vol.256,p.495 - 497, 1975.

In the segment encoding the extracellular domain (hereinafter, also referred to as "extracellular segment"), the nucleotide sequence encoding the antigen binding region includes a nucleotide sequence encoding a light chain variable region and a nucleotide sequence encoding a heavy chain variable region. The nucleotide sequence encoding the antigen binding region may include a nucleotide sequence encoding a part or all of the constant region. The constant region may be either the constant region of the heavy chain or the constant region of the light chain, but is preferably the constant region of the light chain. In the nucleotide sequence encoding the antigen binding region, the order of the nucleotide sequence encoding the light chain variable region and the nucleotide sequence encoding the heavy chain variable region is not particularly limited. For example, the nucleotide sequence encoding the antigen binding region may include, in order from the 5' end, a nucleotide sequence encoding a light chain variable region and a nucleotide sequence encoding a heavy chain variable region. Alternatively, the nucleotide sequence encoding the antigen binding region may include, in order from the 5' end, a nucleotide sequence encoding the heavy chain variable region and a nucleotide sequence encoding the light chain variable region. When a nucleotide sequence encoding a part or all of the constant region of the light chain is included, the nucleotide sequence preferably follows the nucleotide sequence encoding the light chain variable region. When a nucleotide sequence encoding a part or all of the constant region of the heavy chain is included, the nucleotide sequence preferably follows the nucleotide sequence encoding the heavy chain variable region.

The segment encoding the antigen binding region includes or consists of a nucleotide sequence encoding a single-chain antibody that binds to any one of the antigens of the tumor cell. Preferably, the segment encoding the antigen binding region includes or consists of a nucleotide sequence encoding a complex of a MAGE-A4 derived peptide and HLA-A2, a complex of a PRAME derived peptide and HLA-A24, a single-chain antibody that binds CD19 or CEA.

Preferably, the nucleotide sequence encoding a single-chain antibody that binds to a complex of a MAGE-A4 derived peptide and HLA-A2 includes the nucleotide sequence set forth in SEQ ID NOs: 153 to 158. SEQ ID NO: 153 denotes the nucleotide sequence encoding HCDR1; SEQ ID NO: 154 denotes the nucleotide sequence encoding HCDR2; SEQ ID NO: 155 denotes the nucleotide sequence encoding HCDR3; SEQ ID NO: 156 denotes the nucleotide sequence encoding LCDR1; SEQ ID NO: 157 denotes the nucleotide sequence encoding LCDR2; and SEQ ID NO: 158 denotes the nucleotide sequence encoding LCDR3. A specific example of a nucleotide sequence encoding a single-chain antibody including SEQ ID NOs: 153 to 158 is shown in SEQ ID NO: 159. A nucleotide sequence encoding a single-chain antibody in which FR3 is modified based on SEQ ID NO: 159 is shown in, for example, SEQ ID NOs: 160 to 169.

Preferably, the nucleotide sequence encoding a single-chain antibody that binds to a complex of a PRAME derived peptide and HLA-A24 includes the nucleotide sequence set forth in SEQ ID NOs: 170 to 175. SEQ ID NO: 170 is a nucleotide sequence encoding HCDR1; SEQ ID NO: 171 is a nucleotide sequence encoding HCDR2; SEQ ID NO: 172 is a nucleotide sequence encoding HCDR3; SEQ ID NO: 173 is a nucleotide sequence encoding LCDR1; SEQ ID NO: 174 is a nucleotide sequence encoding LCDR2; and SEQ ID NO: 175 is a nucleotide sequence encoding LCDR3. A specific example of a nucleotide sequence encoding a single-chain antibody including SEQ ID NOs: 170 to 175 is shown in SEQ ID NO: 176. A nucleotide sequence encoding a single-chain antibody in which FR3 is modified based on SEQ ID NO: 176 is shown in, for example, SEQ ID NOs: 177 to 179.

Preferably, the nucleotide sequence encoding a single-chain antibody that binds to CD19 includes the nucleotide sequence set forth in SEQ ID NOs: 180 to 185. SEQ ID NO: 180 is a nucleotide sequence encoding HCDR1; SEQ ID NO: 181 is a nucleotide sequence encoding HCDR2; SEQ ID NO: 182 is a nucleotide sequence encoding HCDR3; SEQ ID NO: 183 is a nucleotide sequence encoding LCDR1; SEQ ID NO: 184 is a nucleotide sequence encoding LCDR2; and SEQ ID NO: 185 is a nucleotide sequence encoding LCDR3. A specific example of a nucleotide sequence encoding a single-chain antibody including SEQ ID NOs: 180 to 185 is shown in SEQ ID NO: 186. A nucleotide sequence encoding a single-chain antibody in which FR3 is modified based on SEQ ID NO: 186 is shown in, for example, SEQ ID NOs: 187 to 205.

The nucleotide sequence encoding the single-chain antibody preferably includes a nucleotide sequence encoding a peptide linker between the nucleotide sequence encoding the light chain variable region and the nucleotide sequence encoding the heavy chain variable region. In a single-chain antibody, a peptide linker is a moiety linking a light chain variable region and a heavy chain variable region. The amino acid sequence of the peptide linker is not particularly limited, but generally, a sequence having a length of 15 to 20 amino acids including repetition of an amino acid sequence consisting of a glycine residue and a serine residue (for example, GGGGS: SEQ ID NO: 206) is used. For example, the nucleotide sequence encoding the single-chain antibody may include, in order from the 5' end, a nucleotide sequence encoding a light chain variable region, a nucleotide sequence encoding a peptide linker, and a nucleotide sequence encoding a heavy chain variable region. Alternatively, the nucleotide sequence encoding the single-chain antibody may include, in order from the 5' end, a nucleotide sequence encoding a heavy chain variable region, a nucleotide sequence encoding a peptide linker, and a nucleotide sequence encoding a light chain variable region.

In the CAR, the transmembrane domain is a site for immobilizing the CAR on the cell membrane of the immune cell. The transmembrane domain of the CAR may be derived from a transmembrane protein. For example, in the nucleic acid molecule of the present embodiment, a segment having a nucleotide sequence encoding a transmembrane domain (hereinafter, also referred to as "transmembrane segment") may include a nucleotide sequence encoding the full length of a transmembrane protein. Alternatively, a transmembrane segment may include a nucleotide sequence encoding a part of a transmembrane protein as long as the function as a transmembrane domain is maintained. A part of the transmembrane protein preferably includes all or a part of the transmembrane region of the protein. The transmembrane protein is not particularly limited, and examples thereof include an α chain of a T cell receptor, a β chain of a T cell receptor, CD3ε, CD3ζ, CD4,CD5,CD8 α, CD9,CD16,CD22,CD28,CD33,CD37,CD45,CD64,CD80,CD86,CD 134, CD154,4-1BB (also referred to as CD137), ICOS (Inducible T-cell co-stimulator), and GITR (Glucocorticoid-induced TNF receptor). Preferably, the transmembrane segment includes a nucleotide sequence encoding a transmembrane region of any one of the proteins selected from the group of these transmembrane proteins. Among them, a nucleotide sequence encoding the transmembrane region of CD8α or CD28 is particularly preferable. A specific example of a nucleotide sequence encoding the transmembrane region of CD28 is shown in SEQ ID NO: 207.

The nucleic acid molecule of the present embodiment preferably includes a segment encoding a hinge domain (hereinafter, also referred to as "hinge segment") between the nucleotide sequence encoding the antigen binding region and the transmembrane segment. In the CAR, the hinge domain may confer a length for the antigen binding region to access the antigen and flexibility to avoid steric hindrance to the extracellular domain. The hinge domain is also referred to as a spacer region. The hinge domain of the CAR can be derived from, for example, a transmembrane protein or IgG. Specifically, in the nucleic acid molecule of the present embodiment, the hinge segment may include a nucleotide sequence encoding the entire extracellular region of the transmembrane protein or the entire constant region of IgG. Alternatively, the hinge segment may include a nucleotide sequence encoding a part of the extracellular region of a transmembrane protein or a part of the constant region of an IgG as long as the function as a hinge domain is maintained. Of the extracellular region of the membrane protein, a portion that can be used as a hinge domain is hereinafter also referred to as a "hinge region". Preferably, the hinge segment includes a nucleotide sequence encoding any one region selected from the group consisting of the constant region of the light chain of IgG, the hinge region of CD8α, and the hinge region of CD28. The IgG is preferably IgG4. A specific example of a nucleotide sequence encoding the constant region of the light chain of IgG is shown in SEQ ID NO: 208. A specific example of a nucleotide sequence encoding the CD28 hinge region is shown in SEQ ID NO: 209.

In the CAR, the intracellular domain includes a signaling domain. The signaling domain of the CAR is a site for inducing signaling that activates an immune cell expressing the CAR when the antigen binding region binds to an antigen. In the nucleic acid molecule of the present embodiment, the segment encoding the intracellular domain (hereinafter, also referred to as "intracellular segment") may include a nucleotide sequence encoding a signaling domain. The signaling domain of the CAR may be derived from a membrane protein having an intracellular region, such as a cell membrane receptor, a transmembrane protein, or the like. A signaling domain may be present in the intracellular region of the membrane protein. That is, the intracellular domain of the CAR may include a signaling domain of a membrane protein. Thus, the intracellular segment may include a nucleotide sequence encoding the full length of the membrane protein. Alternatively, the intracellular segment may include a nucleotide sequence encoding a part of the membrane protein as described above as long as the function as a signaling domain is maintained. A part of the membrane protein preferably includes all or a part of the signaling domain of the protein. The membrane protein is not particularly limited, and examples thereof include CD3ζ, CD3γ, CD3δ, CD3ε, CD5, CD22, CD66d, CD79a, CD79b, FcRγ, and FcRβ. Preferably, the intracellular segment includes a nucleotide sequence encoding a signaling domain of at least one protein selected from the group of these membrane proteins. Among them, CD3ζ is preferable. A specific example of a nucleotide sequence encoding the signaling domain of CD3ζ is shown in SEQ ID NO: 210.

The intracellular segment preferably has a nucleotide sequence encoding a co-stimulatory domain in addition to the nucleotide sequence encoding the signaling domain described above. It is known that the signal from the co-stimulatory domain is transmitted to an immune cell (particularly T cell) together with the signal from the signaling domain, thereby improving the proliferation capability, cytotoxic activity, survival rate, and the like of the immune cell. The co-stimulatory domain of the CAR may be derived from a membrane protein having an intracellular region, such as a cell membrane receptor, a transmembrane protein, or the like. A co-stimulatory domain may be present in the intracellular region of the membrane protein. That is, the intracellular domain of the CAR may include a co-stimulatory domain of a membrane protein. Thus, the intracellular segment may include a nucleotide sequence encoding the full length of the membrane protein. Alternatively, the intracellular segment may include a nucleotide sequence encoding a part of the membrane proteins described above as long as the function as a co-stimulatory domain is maintained. A part of the membrane protein preferably includes all or a part of the co-stimulatory domain of the protein. The membrane protein is not particularly limited, and examples thereof include 4-1BB (also referred to as CD137), CD28,GITR,CD2,CD5,CD8,CD9,CD16,CD22,CD33,CD37,CD64,CD80,CD86,CD 134, CD 154, and ICOS. Preferably, the intracellular segment includes a nucleotide sequence encoding a co-stimulatory domain of at least one protein selected from the group of these membrane proteins. Among them, at least one selected from the group consisting of 4-1BB, CD28, and GITR is preferable. A specific example of a nucleotide sequence encoding the co-stimulatory domain of CD28 is shown in SEQ ID NO: 211. A specific example of a nucleotide sequence encoding the co-stimulatory domain of GITR is shown in SEQ ID NO: 212.

In the intracellular domain segment, the order of the nucleotide sequence encoding the signaling domain and the nucleotide sequence encoding the co-stimulatory domain is not particularly limited. For example, the nucleotide sequence encoding the intracellular domain may include, in order from the 5' end, a nucleotide sequence encoding the signaling domain and a nucleotide sequence encoding the co-stimulatory domain. Alternatively, the nucleotide sequence encoding the intracellular domain may include, in order from the 5' end, a nucleotide sequence encoding the co-stimulatory domain and a nucleotide sequence encoding the signaling domain.

The nucleic acid molecule of the present embodiment may include various nucleotide sequences in addition to the nucleotide sequence encoding the CAR as necessary. Examples of such a nucleotide sequence include a leader sequence, a recognition sequence of a restriction enzyme, a nucleotide sequence encoding a peptide tag, a stop codon, and the like. The peptide tag can be appropriately selected from known peptide tags such as a histidine tag, a glutathione-S-transferase (GST) tag, and a FLAG (registered trademark) tag.

A further embodiment of the present invention is described in the above section 1. The present invention relates to a vector including the molecule of a nucleic acid. Specifically, the vector of the present embodiment may be in a form in which the nucleic acid molecule of the present embodiment is incorporated into a known vector. The type of vector is not particularly limited, and examples thereof include a plasmid vector and a viral vector. The vector may be linear or circular. The type of plasmid vector is not particularly limited, and examples thereof include an expression vector, a vector for producing a viral vector, a transposon vector, and a cloning vector. An expression vector is a vector that enables the expression of a protein encoded by the nucleotide sequence of a nucleic acid molecule incorporated into the vector to be expressed in an appropriate host cell such as a mammalian cell, an insect cell, a yeast, or an E. coli. The transposon vector is a vector that enables the incorporation of the nucleic acid molecule incorporated into a transposon vector into the genome of a host cell by being introduced into an appropriate host together with an expression vector in which a gene encoding a transposase is incorporated.

The type of viral vector is not particularly limited, and examples thereof include a retroviral vector, a lentiviral vector, an adenoviral vector, an adeno-associated virus (AAV) vector, a vaccinia virus vector, and an Epstein-Barr virus (EBV) vector. The viral vector is preferably defective in replication capacity so that the virus does not self-replicate in the infected cell.

The vector may include appropriate control sequences as necessary. Examples of such a control sequence include a promoter sequence, an operator sequence, an enhancer sequence, a nucleotide sequence encoding a drug resistance marker, a multiple cloning site, and the like.

As described above, the nucleic acid molecule of the present embodiment may be either DNA or RNA. DNA is a stable substance compared to RNA, and various DNA vectors are commercially available. Therefore, the nucleic acid molecule of the present embodiment which is DNA is advantageous in that it is easy to store and handle. It is known that when RNA encoding a protein is introduced into a cell, the protein can be expressed without being affected by a transcriptional regulatory process. Therefore, the nucleic acid molecule of the present embodiment which is RNA is advantageous in that rapid expression of the CAR is enabled in the immune cell.

### 2. CAR

A further embodiment of the present invention relates to a CAR. The CAR of the present embodiment is the above section 1. A protein molecule encoded by the nucleotide sequence of the nucleic acid molecule described above. The CAR of the present embodiment is a protein produced by a genetic engineering technique including an extracellular domain, a transmembrane domain, and an intracellular domain in this order from the N-terminus. The extracellular domain includes an antigen binding region including a light chain variable region and a heavy chain variable region. The CAR of present embodiment is characterized in that at least three amino acid residues are basic amino acid residues in the light chain FR3 of the antigen binding region. That is, the CAR of the present embodiment includes an antigen binding region in which at least three amino acid residues of the light chain FR3 are basic amino acid residues. The antigen binding region is the same as the modified antigen binding region described above. Hereinafter, the antigen binding region of the CAR of the present embodiment is also referred to as a "modified antigen binding region". The CAR of the present embodiment can also be said to be a mutant of the CAR having the original antigen binding region. The CAR of the present embodiment is preferably expressed in an immune cell and immobilized on a cell membrane of the immune cell. In the immune cell expressing the CAR, production of a cytokine is suppressed as compared with the immune cell expressing the CAR including the original antigen binding region. Preferably, the cytotoxicity of the immune cell on the tumor cell is maintained, and more preferably, the cytotoxicity is improved.

In the CAR of present embodiment, at least three basic amino acid residues of the light chain FR3 are derived from the modification of a codon. In the light chain FR3 of the modified antigen binding region, the at least three basic amino acid residues derived from the modification of a codon may be all arginine residues or all lysine residues. Alternatively, in at least three basic amino acid residues derived from modification of a codon, some of which may be arginine residues and the remainder may be lysine residues.

The amino acid residues before at least three amino acid residues in the light chain FR3 are converted into basic amino acid residues is neutral amino acid residues or acidic amino acid residues, preferably neutral amino acid residues. That is, at least three basic amino acid residues in the light chain FR3 of the modified antigen binding region are residues changed from at least three residues selected from a neutral amino acid residue and/or an acidic amino acid residue in the light chain FR3 of the original antigen binding region.

In the light chain FR3 of the modified antigen binding region, the number of at least three basic amino acid residues derived from modification of a codon is, for example, 3, 4, 5, 6, 7, 8, 9, 10, 11, 12, 13, or 14. The number of at least three basic amino acid residues derived from modification of a codon in the light chain FR3 of the modified antigen binding region is preferably 3 or more and 6 or less, and more preferably 3 or more and 5 or less. As described above, it is preferable that at least three basic amino acid residues in the light chain FR3 of the modified antigen binding region are amino acid residues selected from the positions 57, 58, 59, 60, 61, 62, 63, 65, 67, 70, 72, 74, 76, 77, 79, 80 and 81 of the light chain variable region.

In the modified antigen binding region, at least three amino acid residues selected from the group consisting of the positions 60, 63, 65, 67, 70, 72, 74, 76, 77, 79 and 81 of the light chain variable region are preferably basic amino acid residues derived from modification of a codon. More preferably, three or more and six or less amino acid residues selected from the above group are basic amino acid residues. Particularly preferably, three or more and five or less amino acid residues selected from the above group are basic amino acid residues. For example, the amino acid residue described in any one of the following 1) to 21) is a basic amino acid residue derived from modification of a codon.

1) Amino acid residues at positions 60, 63 and 65 of the light chain variable region;
2) Amino acid residues at positions 60, 63 and 76 of the light chain variable region;
3) Amino acid residues at positions 60, 74 and 76 of the light chain variable region;
4) Amino acid residues at positions 63, 65 and 67 of the light chain variable region;
5) Amino acid residues at positions 63, 65 and 70 of the light chain variable region;
6) Amino acid residues at positions 63, 65 and 72 of the light chain variable region;
7) Amino acid residues at positions 63, 67 and 70 of the light chain variable region;
8) Amino acid residues at positions 63, 67 and 72 of the light chain variable region;
9) Amino acid residues at positions 63, 70 and 72 of the light chain variable region;
10) Amino acid residues at positions 65, 67 and 70 of the light chain variable region;
11) Amino acid residues at positions 65, 67 and 72 of the light chain variable region;
12) Amino acid residues at positions 65, 70 and 72 of the light chain variable region;
13) Amino acid residues at positions 67, 70 and 72 of the light chain variable region;
14) Amino acid residues at positions 70, 72 and 74 of the light chain variable region;
15) Amino acid residues at positions 77, 79 and 81 of the light chain variable region;
16) Amino acid residues at positions 63, 65, 67 and 70 of the light chain variable region;
17) Amino acid residues at positions 63, 65, 67 and 72 of the light chain variable region;
18) Amino acid residues at positions 63, 65, 70 and 72 of the light chain variable region;
19) Amino acid residues at positions 63, 67, 70 and 72 of the light chain variable region;
20) Amino acid residues at positions 65, 67, 70 and 72 of the light chain variable region; and
21) Amino acid residues at positions 63, 65, 67, 70 and 72 of the light chain variable region.

In the modified antigen binding region, at least three amino acid residues selected from the group consisting of positions 63, 65, 67, 70 and 72 of the light chain variable region and at least one amino acid residue selected from the group consisting of positions 60, 74, 76, 77, 79 and 81 of the light chain variable region are preferably basic amino acid residues.

In the light chain FR3 of the modified antigen binding region, when three or more and six or less amino acid residues are basic amino acid residues, the three or more and six or less amino acid residues preferably include three, four, or five amino acid residues selected from the group consisting of positions 63, 65, 67, 70, and 72 of the light chain variable region and one, two, or three amino acid residues selected from the group consisting of positions 60, 74, 76, 77, 79, and 81 of the light chain variable region.

In the light chain FR3 of the modified antigen binding region, when three or more and five or less amino acid residues are basic amino acid residues, the three or more and five or less amino acid residues preferably include three or four amino acid residues selected from the group consisting of positions 63, 65, 67, 70, and 72 of the light chain variable region and one or two amino acid residues selected from the group consisting of positions 60, 74, 76, 77, 79, and 81 of the light chain variable region.

In the modified antigen binding region, at least three amino acid residues selected from the group consisting of positions 60, 74, 67, 79 and 81 of the light chain variable region and at least one amino acid residue selected from the group consisting of positions 63, 65, 67, 70 and 72 of the light chain variable region are preferably basic amino acid residues.

In the light chain FR3 of the modified antigen binding region, when three or more and six or less amino acid residues are basic amino acid residues, the three or more and six or less amino acid residues preferably include three, four, or five amino acid residues selected from the group consisting of positions 60, 74, 76, 77, 79, and 81 of the light chain variable region and one, two, or three amino acid residues selected from the group consisting of positions 63, 65, 67, 70, and 72 of the light chain variable region.

In the light chain FR3 of the modified antigen binding region, when three or more and five or less amino acid residues are basic amino acid residues, the three or more and five or less amino acid residues preferably include three or four amino acid residues selected from the group consisting of positions 60, 74, 76, 77, 79, and 81 of the light chain variable region and one or two amino acid residues selected from the group consisting of positions 63, 65, 67, 70, and 72 of the light chain variable region.

In the modified antigen binding region, when an amino acid residue other than the positions 60, 63, 65, 67, 70, 72, 74, 76, 77, 79 and 81 amino acid residues of the light chain variable region are modified, the modified amino acid residue is preferably an amino acid residue at a site selected from the group consisting of positions 57, 58, 59, 61, and 62.

In the CAR of present embodiment, the extracellular domain includes an antigen binding region including a light chain variable region and a heavy chain variable region. Examples of the antigen binding region include a single-chain antibody. The antigen binding region may include a part or all of the constant regions of the antibody. The constant region may be either the constant region of the heavy chain or the constant region of the light chain, but is preferably the constant region of the light chain. **In** the antigen binding region, the order of the light chain variable region and the heavy chain variable region is not particularly limited. For example, the antigen binding region may include a light chain variable region and a heavy chain variable region in order from the N-terminus. Alternatively, the antigen binding region may include a heavy chain variable region and a light chain variable region in order from the N-terminus. **In** the antigen binding region, a part or all of the constant region of the light chain is preferably included after the light chain variable region. In the antigen binding region, a part or all of the constant region of the heavy chain is preferably included after the heavy chain variable region.

In the CAR of present embodiment, the antigen binding region may include or consist of a single-chain antibody that binds to any one of the antigen present on the surface of the tumor cell and the antigen present in the tumor cell. Preferably, the antigen binding region includes or consists of a single-chain antibody that binds to a complex of a MAGE-A4 derived peptide and HLA-A2, a complex of a PRAME derived peptide and HLA-A24, CD19, or CEA.

Preferably, the amino acid sequence of the single-chain antibody that binds to the complex of the MAGE-A4 derived peptide and HLA-A2 includes the amino acid sequence set forth in SEQ ID NOs: 213 to 218. SEQ ID NO: 213 is the amino acid sequence of HCDR1, SEQ ID NO: 214 is the amino acid sequence of HCDR2, SEQ ID NO: 215 is the amino acid sequence of HCDR3, SEQ ID NO: 216 is the amino acid sequence of LCDR1, SEQ ID NO: 217 is the amino acid sequence of LCDR2, and SEQ ID NO: 218 is the amino acid sequence of LCDR3. A specific example of the amino acid sequence of a single-chain antibody including SEQ ID NOs: 213 to 218 is shown in SEQ ID NO: 219. The amino acid sequence of the single-chain antibody in which FR3 is modified based on SEQ ID NO: 219 is shown in, for example, SEQ ID NOs: 220 to 229.

Preferably, the amino acid sequence of the single-chain antibody that binds to the complex of the PRAME derived peptide and HLA-A24 includes the amino acid sequence set forth in SEQ ID NOs: 230 to 235. SEQ ID NO: 230 is the amino acid sequence of HCDR1, SEQ ID NO: 231 is the amino acid sequence of HCDR2, SEQ ID NO: 232 is the amino acid sequence of HCDR3, SEQ ID NO: 233 is the amino acid sequence of LCDR1, SEQ ID NO: 234 is the amino acid sequence of LCDR2, and SEQ ID NO: 235 is the amino acid sequence of LCDR3. A specific example of the amino acid sequence of a single-chain antibody including SEQ ID NOs: 230 to 235 is shown in SEQ ID NO: 236. The amino acid sequence of the single-chain antibody in which FR3 is modified based on SEQ ID NO: 236 is shown in, for example, SEQ ID NOs: 237 to 239.

Preferably, the amino acid sequence of the single-chain antibody that binds to CD19 includes the amino acid sequence set forth in SEQ ID NOs: 240 to 245. SEQ ID NO: 240 is the amino acid sequence of HCDR1, SEQ ID NO: 241 is the amino acid sequence of HCDR2, SEQ ID NO: 242 is the amino acid sequence of HCDR3, SEQ ID NO: 243 is the amino acid sequence of LCDR1, SEQ ID NO: 244 is the amino acid sequence of LCDR2, and SEQ ID NO: 245 is the amino acid sequence of LCDR3. A specific example of the amino acid sequence of a single-chain antibody including SEQ ID NOs: 240 to 245 is shown in SEQ ID NO: 246. The amino acid sequence of the single-chain antibody in which FR3 is modified based on SEQ ID NO: 246 is shown in, for example, SEQ ID NOs: 247 to 265.

The single-chain antibody preferably includes a peptide linker between the light chain variable region and the heavy chain variable region. The peptide linker is as described above. For example, the single-chain antibody may include a light chain variable region, a peptide linker, and a heavy chain variable region in order from the N-terminus. Alternatively, the single-chain antibody may include a heavy chain variable region, a peptide linker, and a light chain variable region in order from the N-terminus.

In the CAR of present embodiment, the transmembrane domain may include the full length of the transmembrane protein. Alternatively, the transmembrane domain may include a part of a transmembrane protein as long as its function is maintained. A part of the transmembrane protein preferably includes all or a part of the transmembrane region of the protein. Examples of the transmembrane protein that can be used for the transmembrane domain include the above section 1. Examples thereof include the proteins exemplified above. Preferably, the transmembrane domain is, for example, a transmembrane region of any one protein selected from the group consisting of an α chain of a T cell receptor, a β chain of a T cell receptor, CD3ε, CD3ζ, CD4,CD5,CD8 α, CD9,CD16,CD22,CD28,CD33,CD37,CD45,CD64,CD80,CD86,CD 134, CD154,4-1BB (also referred to as CD137), ICOS, and GITR. Among them, the transmembrane region of CD8α or CD28 is preferable. A specific example of the amino acid sequence of the transmembrane region of CD28 is shown in SEQ ID NO: 266.

The CAR of the present embodiment preferably includes a hinge domain between the antigen binding region and the transmembrane domain. The hinge domain may include the entire extracellular region of the transmembrane protein or the entire constant region of IgG. Alternatively, a hinge domain may include a part of the extracellular region of a transmembrane protein or a part of the constant region of an IgG as long as its function is maintained. Examples of the protein that can be used for the hinge domain include the above section 1. Examples thereof include the proteins exemplified above. Preferably, the hinge domain is, for example, any one region selected from the group consisting of a constant region of a light chain of IgG, a hinge region of CD8α, and a hinge region of CD28. The IgG is preferably IgG4. A specific example of the amino acid sequence of the constant region of the light chain of IgG is shown in SEQ ID NO: 267. A specific example of the amino acid sequence of the CD28 hinge region is shown in SEQ ID NO: 268.

In the CAR of present embodiment, the intracellular domain may include the full length of a membrane protein having an intracellular region. Alternatively, the intracellular domain may include a part of the membrane protein described above as long as the function as a signaling domain is maintained. The signaling domain may be present in the intracellular region of the membrane protein. A part of the membrane protein having the intracellular region preferably includes all or a part of the signaling domain of the protein. Examples of the membrane protein that can be used for the intracellular domain include the above section 1. Examples thereof include the proteins exemplified above. Preferably, the intracellular domain includes, for example, a signaling domain of at least one protein selected from the group consisting of CD3ζ, CD3γ, CD3δ, CD3ε, CD5, CD22, CD66d, CD79a, CD79b, FcRγ and FcRβ. Among them, the signaling domain of CD3ζ is preferred. A specific example of the amino acid sequence of the signaling domain of CD3ζ is shown in SEQ ID NO: 269.

In the CAR of present embodiment, the intracellular domain preferably has a co-stimulatory domain in addition to the signaling domain. The intracellular domain may include the full length of a membrane protein having an intracellular region. Alternatively, the intracellular domain may include a part of a membrane protein as described above as long as the function as a co-stimulatory domain is maintained. The co-stimulatory domain may be present in the intracellular region of each of the above proteins. A part of the membrane protein having an intracellular region preferably includes all or a part of the co-stimulatory domain of the protein. Examples of the membrane protein that can be used for the co-stimulatory domain include the above section 1. Examples thereof include the proteins exemplified above. Preferably, the co-stimulatory domain includes a co-stimulatory domain of at least one protein selected from the group consisting of, for example, 4-1BB (also referred to as CD137), CD28,GITR,CD2,CD5,CD8,CD9,CD16,CD22,CD33,CD37,CD64,CD80,CD86,CD 134, CD154, and ICOS. Among them, co-stimulatory domains of 4-1BB, CD28 and GITR are preferred. A specific example of the amino acid sequence of the co-stimulatory domain of CD28 is shown in SEQ ID NO: 270. A specific example of the amino acid sequence of the co-stimulatory domain of GITR is shown in SEQ ID NO: 271.

In the intracellular domain, the order of the signaling domain and the co-stimulatory domain is not particularly limited. For example, the intracellular domain may include a signaling domain and a co-stimulatory domain in order from the N-terminus. Alternatively, the intracellular domain may include a co-stimulatory domain and a signaling domain in order from the N-terminus.

The CAR of the present embodiment may include an additional oligopeptide or polypeptide as necessary. Examples of such a oligopeptide and a polypeptide include a signal peptide, a peptide tag, and the like. The peptide tag can be appropriately selected from known peptide tags such as a histidine tag, a GST tag, and a FLAG (registered trademark) tag.

The CAR of the present embodiment can be produced by a protein expression system using the nucleic acid molecule of the present embodiment. The protein expression system may be an expression system using a host cell or a cell-free protein synthesis system. In the generation by an expression system using a host cell, for example, the CAR of the present embodiment can be expressed by introducing the nucleic acid molecule of the present embodiment incorporated into an expression vector suitable for the host cell to be used into the host cell. Examples of the cell-free protein synthesis system include a wheat germ derived synthesis system, an E. coli derived synthesis system, and a reconfigurable cell-free protein synthesis system. When the CAR is produced in the host cell, the host cell may be lysed with a solution including a suitable solubilizing agent to release the CAR in the solution. In the cell-free protein synthesis system, the synthesized CAR is included in the reaction solution. The CAR released in the liquid can be recovered by a known method such as column chromatography. For example, when the produced CAR has a histidine tag or a GST tag as a peptide tag, the peptide can be recovered by affinity chromatography using a carrier including Ni-NTA (nitrilotriacetic acid that forms a chelate with a nickel ion) or glutathione. The recovered CAR may be purified by a known method such as gel filtration or dialysis, as necessary.

### 3. Immune cell comprising CAR

A further embodiment of the invention relates to an immune cell comprising the CAR. The immune cell comprising the CAR of present embodiment is an immune cell expressing the CAR of the section 2. above by introducing the nucleic acid molecule of the section 1. above The details of the CAR and the nucleic acid molecule are as described above. The examples of the immune cell before the nucleic acid molecule is introduced include an immune cell collected from a mammal including a human, an immune cell prepared from a stem cell, and an immune cell established as a cell line. Specifically, an immune cell obtained by leukapheresis, an immune cell isolated from blood, and the like are exemplified.

Examples of the immune cell separated or isolated from the biological sample include peripheral blood mononuclear cells (PBMC), T cells, and natural killer (NK) cells. Among them, T cells and NK cells are preferred. T cells include CD8 positive T cells, CD4 positive T cells, cytotoxic T cells, helper T cells, regulatory T cells, and tumor-infiltrating lymphocytes. Preferred T cells are CD8 positive T cells and cytotoxic T cells. The cell line of immune cells is preferably a cell line derived from lymphocytes, and examples thereof include Jurkat cells, MOLT-4 cells, and U-937 cells. Hereinafter, the T cell expressing the CAR may be referred to as a "CAR-T cell".

The method for introducing the nucleic acid molecule of the section 1. above into an immune cell is not particularly limited, and can be appropriately selected from known gene introduction methods. Examples of the gene introduction method include gene introduction by a lipofection method, an electroporation method, a calcium phosphate method, a cationic polymer, gene introduction by a viral vector, and gene introduction by a transposon. In gene introduction by the lipofection method and a cationic polymer, commercially available transfection reagents such as FuGENE (registered trademark) and JetPEI (registered trademark) may be used.

The immune cell including the CAR of present embodiment can be cultured in the same manner as the immune cell before the nucleic acid molecule of present embodiment is introduced. The culture medium, serum, additive, and the like can be appropriately determined according to the type of immune cell to be used. Examples of the culture medium include MEM, DMEM, and RPMI-1640. When the immune cell is a lymphocyte, for example, a commercially available lymphocyte medium such as GT-T502, GT-T503, or GT-T551 (Takara Bio Inc.) may be used. Examples of the serum include a fetal bovine serum (FBS) and a human type AB serum. Examples of the additive include L-glutamine, insulin, and IL-2. Examples of the culture conditions for immune cells include conditions under a 5% CO₂ atmosphere at 37 ° C.

In the immune cell including the CAR of the present embodiment, the CAR is expressed as a transmembrane protein. When the extracellular domain of the CAR binds to an antigen of a tumor cell, the immune cell is activated by a signal from the intracellular domain of the CAR. Then, the immune cell including the activated CAR of present embodiment exerts a cytotoxicity by release of a cytotoxic protein (for example, perforin and granzyme) and an antitumor cytokine (for example, tumor necrosis factor (TNF)-α, lymphokine) and expression of a cell surface molecule that induces cell death such as a Fas ligand. Preferably, the immune cell including the CAR of the present embodiment has a maintained cytotoxicity and more preferably has an improved cytotoxicity as compared with the immune cell including the CAR having the original antigen binding region. The cytotoxicity by the immune cell including the CAR can be examined by a known method such as a cytotoxicity assay. Specifically, the method of Example 3 described below is illustrated. As in Example 4 described later, the cytotoxicity can also be evaluated by administering the immune cell including the CAR to a non-human animal transplanted with a tumor cell and measuring the size of the tumor. On the other hand, in the immune cell including the CAR of the present embodiment, the release amount of a cytokine (for example, IFNγ, granulocyte macrophage colony stimulating factor (GM-CSF)) that causes CRS is reduced as compared with the immune cell including the CAR having the original antigen binding region. The amount of cytokine released by the immune cell including the CAR can be examined by a known method such as enzyme-linked immunosorbent assay (ELISA). Specifically, the method of Example 5 described below is illustrated.

### 4. Method for producing immune cell including car

A further embodiment of the present invention relates to a method for producing an immune cell including the CAR (hereinafter, also referred to as "production method of the present embodiment"). In the production method of the present embodiment, the above section 1. A nucleic acid molecule of the present invention is introduced into an immune cell, and the immune cell is transformed into the immune cell as described in the above section 2. By expressing the CAR of the above section 3. An immune cell including the CAR of the CAR is produced. The details of the CAR and the nucleic acid molecule are as described above. Further, the above section 1. Details of the immune cell before the nucleic acid molecule of the nucleic acid is introduced are as described above. PBMCs, T cells and NK cells are preferred. When the immune cell including the CAR obtained by the production method of the present embodiment is transplanted into a living body, it is preferable to use an immune cell separated or isolated from a biological sample collected from the living body itself or another living body of the same kind as the living body.

The method for introducing the nucleic acid molecule of the section 1. above into the immune cell is as described above. After the nucleic acid molecule is introduced into the immune cell, the immune cell is preferably cultured for a predetermined period. The method for culturing the immune cell is as described above. The predetermined period of time can be at least a period of time until the CAR is expressed in the immune cell. The period until the CAR is expressed is determined depending on the method for introducing a nucleic acid molecule, and is, for example, 3 hours or more and 72 hours or less, preferably 6 hours or more and 48 hours or less. When the method for introducing the nucleic acid molecule is a method for enabling stable expression of the CAR in the immune cell, the predetermined period may be a period sufficient for the immune cell expressing the CAR to proliferate. Such a period is not particularly limited, but is, for example, 48 hours or more and 20 days or less, preferably 72 hours or more and 14 days or less.

By introducing the nucleic acid molecule of the section 1. above into an immune cell, the CAR of the section 2. is expressed on the immune cell. It may be confirmed that the CAR is expressed in the immune cell as necessary. The expression of the CAR in the immune cell may be confirmed by a known protein detection method such as ELISA, flow cytometry, immunoprecipitation, polyacrylamide gel electrophoresis, or Western blotting.

### 5. Pharmaceutical composition

A further embodiment of the present invention relates to a pharmaceutical composition for treating a malignant tumor including the immune cell including the CAR (hereinafter, also referred to as "pharmaceutical composition of the present embodiment"). The pharmaceutical composition of the present embodiment includes as an active ingredient the immune cell comprising the CAR of the section 3. Details of the CAR and the immune cell including the CAR are as described above. The pharmaceutical composition of the present embodiment may further include a pharmaceutically acceptable additive. Examples of such additives include aqueous media for stably preserving immune cells, D-glucose, dextran, serum albumin, and dimethyl sulfoxide (DMSO). Examples of the aqueous medium include physiological saline, phosphate buffered saline (PBS), and a composite electrolyte solution. The pharmaceutical composition of the present embodiment is preferably administered parenterally to a patient. That is, the pharmaceutical composition is preferably in a form suitable for parenteral administration, for example, in the form of an injection, and an infusion solution.

The malignant tumor to be treated by the pharmaceutical composition of the present embodiment is a tumor including a tumor cell having an antigen recognized by the CAR. The malignant tumor may be a blood cancer or a solid cancer. Examples thereof include acute leukemia (acute myeloid leukemia, B-cell acute lymphoblastic leukemia, acute monocytic leukemia, acute erythroleukemia, acute megakaryoblastic leukemia, etc.), chronic leukemia (chronic myelogenous leukemia, chronic lymphocytic leukemia, chronic monocytic leukemia, etc.), lymphoma (diffuse large B-cell lymphoma, mantle cell lymphoma, Burkitt's lymphoma, lymphoplasmacytic lymphoma, marginal zone lymphoma, etc.), multiple myeloma, melanoma, breast cancer, prostate cancer, bladder cancer, uterine cancer, liver cancer, spleen cancer, lung cancer, gastric cancer, esophageal cancer, ovarian cancer, osteosarcoma, and neuroblastoma.Kidney cancer, pancreatic cancer, spleen cancer, lung cancer, gastric cancer, esophageal cancer, ovarian cancer, osteosarcoma, neuroblastoma, and the like.

### 6. Treatment method

A further embodiment of the present invention is a method for treating a malignant tumor using the immune cell comprising the CAR of the section 3. (hereinafter, also referred to as "treatment method of the present embodiment"). The method comprises administering the pharmaceutical composition described above to a malignant tumor patient. The malignant tumors to be treated are as described above.

In the administration step, preferably, the immune cell is administered parenterally to the patient. Parenteral administration includes, for example, intravenous, intraarterial, intramuscular, intraperitoneal, and subcutaneous administration. Among them, intravenous administration is preferable.

The administration of the pharmaceutical composition of the present embodiment can be appropriately determined according to the type of cancer, the disease state of the patient, the age, the weight, and the like. For example, for an adult with a body weight of 50 kg or more, the dose is 1×10⁴ cells or more and 1×10¹⁰ cells or less, preferably 1×10 ⁵ cells or more and 1×10⁹ cells or less, and more preferably 1×10⁶ cells or more and 5×10⁸ cells or less as the immune cell including the CAR per administration. The number of times of administration may be administration only once or may be administration a plurality of times. After administration, the number of administrations is usually one because the immune cell including the CAR engrafts and proliferates in the body of the patient. However, when it is determined that engraftment or proliferation of the immune cell is insufficient, the administration may be performed a plurality of times. The administration interval may be, for example, 1 to 4 times per day, every 1 week, every 10 to 30 days, every 1 month, every 3 to 6 months, or every 1 year.

The pharmaceutical composition of the present embodiment can be used in combination with other anticancer agents. Administration of another anticancer agent and the above section 5. The administration of the pharmaceutical composition may be performed on the same day or on different days. Examples of the other anticancer agent include, but are not limited to, alkylating agents such as cyclophosphamide, antimetabolites such as pentostatin, molecular target drugs such as rituximab, kinase inhibitors such as imatinib, proteasome inhibitors such as bortezomib, calcineurin inhibitors such as cyclosporine, anticancer antibiotics such as idarubicin, plant alkaloids such as irinotecan, platinum preparations such as cisplatin, hormone therapy agents such as tamoxifen, and immunomodulatory agents such as nivolumab and pembrolizumab.

The number of white blood cells of the patient may be reduced by performing lymphocyte-depleting chemotherapy as treatment before administration of the pharmaceutical composition of the present embodiment. For example, fludarabine, cyclophosphamide, bendamustine, and the like are used for lymphocyte-depleting chemotherapy.

### 7. Method for reducing cytokine production of immune cell

A further embodiment of the invention relates to a method of reducing cytokine production of an immune cell comprising the CAR (hereinafter, also referred to as a "reduction method of the present embodiment"). The reduction method of the present embodiment comprises preparing he nucleic acid molecule of the section 1. above; and introducing the nucleic acid molecule of the section 1. above and expressing the CAR of the section 2. above on the immune cell. The immune cell comprising the obtained CAR expresses a CAR including a modified antigen binding region. The immune cell expressing the CAR has reduced production of cytokines as compared with the immune cell expressing the CAR including the original antigen binding region. On the other hand, the cytotoxicity by the immune cell expressing the CAR including the modified antigen binding region is maintained or improved as compared with the immune cell expressing the CAR including the original antigen binding region. The reduction method of the present embodiment can contribute to further reduction of side effects caused by cytokines in a method for treating with the immune cell expressing a CAR. Details of the nucleic acid molecule and the CAR are as described above. The method for introducing a nucleic acid molecule into an immune cell and the method for measuring a cytokine produced by an immune cell including the CAR are as described above.

Hereinafter, the present invention is described in detail with reference to Examples, but the present invention is not limited to these Examples.

### EXAMPLES

### Example 1: Generation of CAR-T cells binding to MAGE-A4/HLA-A2 complex and confirmation of their efficacy (1)

### (1) Acquisition of nucleic acid molecule encoding CAR as template

Two plasmid DNAs for production of viral vectors including a gene encoding a CAR having a single-chain antibody that binds to MAGE-A4/HLA-A2 complex were obtained in the same manner as in the Example of US2020/0276237, which is incorporated herein by reference. The complex recognized by the single-chain antibody was a complex of MAGE-A4 and HLA-A*02:01. Hereinafter, the CAR encoded by the gene in one plasmid DNA is referred to as "MAGE-A4-zG", and the CAR encoded by the gene in the other plasmid DNA is referred to as "MAGE-A4-zG-s1". The amino acid sequence of the MAGE-A4 derived peptide in the above MAGE-A4/HLA-A2 complex was GVYDGREHTV (SEQ ID NO: 1). In order to produce a nucleic acid molecule encoding a mutant of MAGE-A4-zG and MAGE-A4-zG-s1 by a PCR method, the above plasmid DNAs were used as templates.

Referring to Fig. 2A, in the gene encoding MAGE-A4-zG, from the 5' end, a leader sequence (Leader), a nucleotide sequence encoding a heavy chain variable region (VH), a nucleotide sequence encoding a linker (L), a nucleotide sequence encoding a light chain variable region (VL), a nucleotide sequence encoding a light chain constant region (CL), a nucleotide sequence encoding a transmembrane domain of CD28 (CD28TM), a nucleotide sequence encoding CD3ζ, and a nucleotide sequence encoding an intracellular domain of GITR (GITRICD) were linked in this order. The VH, L, VL and CL constituted a segment having a nucleotide sequence encoding the extracellular domain of the CAR. The VH, L and VL constituted a segment with a nucleotide sequence encoding a single-chain antibody that specifically binds to MAGE-A4/HLA-A2 complex. CD3ζ and GITRICD constituted a segment with a nucleotide sequence encoding the intracellular domain of the CAR. CL was a segment with a nucleotide sequence encoding the hinge domain of the CAR.

The gene encoding MAGE-A4-zG-s1 had the same configuration as the gene encoding MAGE-A4-zG except that an extra nucleotide sequence was added to the 3' end of the gene encoding MAGE-A4-zG (3' side of GITRICD). As a result of searching in a known database, it was confirmed that the surplus nucleotide sequence did not coincide with the nucleotide sequence encoding any protein. As a result of computer analysis, it was predicted that the amino acid sequence corresponding to the surplus nucleotide sequence forms a linear polypeptide having no secondary structure.

MAGE-A4-zG and MAGE-A4-zG-s1 were CARs including, as extracellular domains, a single-chain antibody consisting of VH, VL and a linker connecting them, and CL, including CD28TM as a transmembrane domain, and including CD3ζ and GITRICD as an intracellular domain.

### (2) Obtainment of a nucleic acid molecule encoding a mutant of MAGE-A4-zG and MAGE-A4-zG-s1

### [Reagents]

QIAprep Spin Miniprep kit (QIAGEN Inc.)
PrimeSTAR (registered trademark) Max Premix (Takara Bio Inc.)
Ligation high ver.2 (Toyobo Co.,Ltd.)
T4 Polynucleotide Kinase (Toyobo Co.,Ltd.)
Dpn I (Toyobo Inc.)
Competent high DH5 α (Toyobo Corporation)

### (2.1) Primer design and PCR

A primer set for obtaining a polynucleotide encoding a CAR including a single-chain antibody in which the amino acid residues shown in a) or b) in MAGE-A4-zG below are substituted with arginine residues was designed based on the nucleotide sequence of SEQ ID NO: 3. A primer set for obtaining a polynucleotide encoding a CAR including a single-chain antibody in which the amino acid residue shown in a) below in MAGE-A4-zG-s1 was substituted with an arginine residue was designed based on the nucleotide sequence of SEQ ID NO: 9.

a) Amino acid residues at positions 63, 65, 67 and 70 of the VL as defined by Kabat; and
b) Amino acid residues at positions 63, 65, 67 and 72 of the VL as defined by Kabat

Each of the above plasmid DNAs including a gene encoding each of MAGE-A4-zG and MAGE-A4-zG-s1 was used as template DNA. A PCR reaction solution including template DNA, a primer set (SEQ ID NOs: 77 and 78) and a PrimeSTAR (registered trademark) Max Premix was prepared, and a PCR reaction was performed. Thus, a PCR product in which a recognition site of a restriction enzyme (hereinafter, referred to as restriction site) was added to the 5' end and the 3' end was obtained. DpnI was added to the resulting PCR product to fragment the template plasmid DNA. Using the DpnI-treated PCR product as a template, a PCR reaction solution including a primer set for producing a mutant and a PrimeSTAR (registered trademark) Max Premix was prepared, and a PCR reaction was performed. The resulting PCR product, plasmid DNA (empty vector) for producing a viral vector subjected to restriction enzyme treatment, Ligation high ver.2, and a T4 polynucleotide kinase were mixed, and incubated at 16 ° C. for 1 hour to perform a ligation reaction.

Hereinafter, CARs having a single-chain antibody in which the amino acid residues shown in a) and b) in MAGE-A4-zG are substituted with arginine residues are referred to as "MAGE-A4-zG-m1" and "MAGE-A4-zG-m2", respectively. Hereinafter, CARs including a single-chain antibody in which the amino acid residue shown in a) in MAGE-A4-zG-s1 is an arginine residue are referred to as "MAGE-A4-zG-m1-s1". Table 1 shows the correspondence between each mutant and the SEQ ID NO. of the primer set for production thereof. In the table, "F" indicates the SEQ ID NO. of the nucleotide sequence of the forward primer, and "R" indicates the SEQ ID NO. of the nucleotide sequence of the reverse primer. The same applies to the table of the following examples.

**[Table 1]**

| NAME 0F CAR | F | R |
|---|---|---|
| MAGE-A4-zG-m1 | 79 | 80 |
| MAGE-A4-zG-m2 | 81 | 82 |
| MAGE-A4-zG-m1-s1 | 79 | 80 |

### (2.2) transformation, extraction of plasmid DNA and sequencing

Using the solution after the ligation reaction and DH5α, a transformant of E. coli was obtained by a heat shock method. A single colony on an agar medium was cultured in an ampicillin-containing LB liquid medium. Plasmid DNAs were extracted from the resulting E. coli using QIAprep Spin Miniprep kit. Each of the resulting plasmid DNAs was sequenced. Sequencing confirmed that a nucleic acid molecule encoding a mutant of MAGE-A4-zG and MAGE-A4-zG-s1 was obtained.

### (3) Generation of T cells expressing MAGE-A4-zG,MAGE-A4-zG-s1 and mutants thereof

### (3.1) Preparation of human lymphocytes

PBMCs were separated from blood provided by healthy donors using Ficoll-Paque (trademark) PLUS (GE Healthcare). The resulting PBMC was used as a human lymphocyte. The collection and analysis of specimens such as human peripheral blood used in the present study were performed according to the Helsinki Declaration, and all were performed with the written consent of the subject in accordance with a protocol approved by the Ethics Committee of the Mie University School of Medicine. The collected specimen was encoded so as not to be identifiable, and stored in a refrigerator and a liquid nitrogen tank subjected to antitheft treatment. The personal information of the subject was anonymized, and strict attention and treatment were performed so that the privacy of the individual and the result of the gene analysis were not leaked to the outside.

### (3.2) Separation and culture of PBMC

Anti-CD3 antibodies OKT-3 (eBioscience) and RetroNectin (registered trademark) (Takara Bio Inc.) were added to the ACD-A solution (Terumo Corporation) at final concentrations of 5 µg/mL and 25 µg/mL, respectively. To each well of a 12 well plate (Nunc), 400 µL of the resulting solution was added, and the plate was left to stand at 4°C overnight. The liquid in each well was removed and washed three times with PBS to prepare a PBMC culture plate. AB serum (300 µL, Veritas) was added to the lymphocyte culture medium GT-T503(50 mL, Takara Bio Inc.), and human IL-2 (NIPRO CORPORATION) was further added so as to have a final concentration of 300 IU/mL to prepare a PBMC culture medium. The PBMCs obtained in the above (3.1) were suspended in the prepared medium at 2.5×10 ⁵ to 3.0×10 ⁵ cells/mL. To each well of the PBMC culture plate, 2 mL of the cell suspension was added, and cultured in a CO ₂ incubator at 37°C for 3 days. On day 3, 1 mL of the medium was replaced, and the cells were cultured for another day.

### (3.3) Introduction of a CAR gene into an immune cell by retrovirus

### (i) Generation of retrovirus by packaging cells plat-A

Plasmid DNAs for producing respective viral vectors including genes encoding MAGE-A4-zG,MAGE-A4-zG-s1 and mutants thereof obtained in the above (2) were introduced into packaging cells plat-A using FuGENE (registered trademark) (Promega K.K.) and cultured for 2 days. Thereafter, the culture supernatant was recovered to obtain a liquid including the viral vector (hereinafter, also referred to as "viral liquid").

### (ii) Preparation of retrovirus diluent and wash solution

To a mixture of PBS (40 mL) and ACD-A solution (2.4 mL), Albuminar (registered trademark) 25% (CSL Behring K.K.) was added to a final concentration of 2.5% to prepare a retrovirus diluent. To PBS (39 mL), 25% of Albuminar (registered trademark) was added so as to have a final concentration of 1.5%, thereby preparing a retroviral washing solution.

### (iii) Preparation of plate for retroviral infection

RetroNectin (registered trademark) (Takara Bio Inc.) was diluted with a retrovirus diluent to a final concentration of 20 µg/mL. To each well of a 24 well plate, 250 µL of a diluent of RetroNectin (registered trademark) was added and the plate was left to stand at 4°C overnight. The liquid in each well was removed and washed twice with retroviral wash. To each well, 1 mL of the viral liquid prepared in the above (3.1) was added, and centrifuged at 2000×g for 2 hours at 32° C. to coat each well with retrovirus. The viral liquid in each well was removed and washed twice with a retroviral washing solution to prepare a plate for retroviral infection.

### (iv) Generation of CAR-T cells by retroviral infection

The PBMCs cultured in the above (3.2) were recovered and suspended in a PBMC culture medium so as to be 1.3×10 ⁵ cells/mL. The cell suspension was added to each well of the plate for retroviral infection in an amount of 1.5 mL, and centrifuged at 1000×g for 10 minutes at 32°C. Thereafter, the plate was left to stand in a CO ₂ incubator, and the cells were cultured at 37° C. Thus, T cells (CAR-T cells) expressing various CARs including MAGE-A4-zG,MAGE-A4-zG-s1 and mutants thereof were obtained. CAR-T cells were used in various assays 12 days after PBMC separation.

### (4) Confirmation of cytotoxicity of CAR-T cells and cytokine secretion amount

### (4.1) Cytotoxicity assay

Using the various CAR-T cells produced in the above (3) as effector cells, the cytotoxicity was measured by an N-SPC (registered trademark) non-RI cytotoxicity assay kit (TECHNO SUZUTA Co.,Ltd.). As target cells, a human melanoma cell line SK-MEL-37 or NW-MEL-38 was used. These target cells were A2 positive MAGE-A4 positive tumor cells.

The measurement principle of the above assay kit was as follows. When the BM-HT Reagent attached to the above assay kit is added to the target cells, the BM-HT Reagent is hydrolyzed by intracellular esterase to generate an HT chelate in the target cells. When the target cells are killed by effector cells, the HT chelate leaks into the culture supernatant. When the Eu Solution attached to the above assay kit is added to the culture supernatant including the HT chelate, the Eu/HT complex is formed. When the complex is excited by laser light, time-resolved fluorescence is generated. Since leakage of HT chelates depends on the cytotoxic activity of effector cells, the cytotoxicity can be quantitatively measured by measuring time-resolved fluorescence.

The target cells were suspended in a medium so as to be 1×10 ⁴ cells/mL, and BM-HT Reagent was added thereto. Effector cells (CAR-T cells) and target cells were mixed at a cell number ratio of 3 : 1 or 1 : 1, added to each well of a 96 well plate, and co-cultured at 37° C. for 2 hours. For comparison, two wells (control wells) to which target cells not mixed with effector cells were added were prepared and cultured in the same manner. At 1.5 hours from the start of culture, Detergent attached to the kit was added to one of the control wells, and the fluorescence detected from the control well was defined as maximum fluorescence. The other of the control wells was untreated, and the fluorescence detected from the control well was defined as minimum fluorescence. Eu Solution (120 µL) was added to the culture supernatant (12 µL) of the co-culture, and the mixture was left to stand at room temperature for 15 minutes, and then time-resolved fluorescence was measured.

### (4.2) Measurement of IFNγ

### (i) Preparation of sample

As effector cells, CAR-T cells including MAGE-A4-zG-s1 produced in the above (3) and CAR-T cells including MAGE-A4-zG-m1-s1 were used. For comparison, the control cells produced in the above (3) were also used. As target cells, SK-MEL-37 and NW-MEL-38 of A2 positive MAGE-A4 positive tumor cells were used. Each of the effector cells and the target cells was suspended in a medium so as to be 1×10 ⁵ cells/mL, added to each well of a 96 well plate, and co-cultured at 37° C. for 12 hours. The culture supernatant was recovered and used as a sample.

### (ii) Preparation of reagent

Invitrogen (registered trademark) Human IFN gamma Uncoated ELISA with Plates (Thermo Fisher Scientific) was used for measuring IFNγ in respective samples. The 10× Coating Buffer was diluted 10 times with purified water to prepare Coating Buffer. Capture antibody (anti-human IFNγ antibody) (48 µL) was added to Coating buffer (12 mL) to prepare a dilute solution of capture antibody. To each well of a 96 well flat bottom plate Costar (registered trademark) 9018 (Corning K.K.), 100 µL of a dilution solution of the capture antibody was added. The plate was left to stand at 4° C. overnight. The solution in the well was removed and the wells were washed five times with 0.05% PBS-T (PBS, 0.05% Tween (trademark)-20). The 5× Assay Diluent was diluted 5 times with purified water to prepare Assay Diluent. To each well, 200 µL of Assay Diluent was added, and the mixture was blocked at room temperature for 1 hour. Assay Diluent in the wells was removed and the wells were washed five times with 0.05% PBS-T to obtain a plate coated with a capture antibody. Recombinant human IFN-γ was dissolved in Assay Diluent to prepare a solution-final concentration of 1000 pg/mL. The solution was diluted 2 times in 7 steps to prepare a standard. Detection antibody (biotin-labeled anti-human IFNγ antibody) (48 µL) was added to Coating buffer (12 mL) to prepare a dilute solution of the detection antibody. Enzyme (streptavidin-HRP) (48 µL) was added to Coating buffer (12 mL) to prepare a dilute solution of the enzyme.

### (iii) Measurement of IFNγ

Each of the sample and standard was added to the wells of the plate and incubated at room temperature for 2 hours. The wells were washed five times with 0.05% PBS-T. To each well, 100 µL of a diluent of the detection antibody was added. The plate was incubated at room temperature for 1 hour. The solution in the well was removed and the well was washed five times with 0.05% PBS-T. To each well, 100 µL of a diluent of the enzyme was added. The plate was incubated at room temperature for 30 minutes. The solution in the well was removed and the well was washed 7 times with 0.05% PBS-T. To each well, 100 µL of a TMB substrate solution was added. The plate was incubated in the dark at room temperature for 15 minutes. 0.18M To each well, 50 µL of the H ₂ SO ₄ was added, and the reaction was stopped, and immediately, the absorbance at 450 nm was measured using a microplate reader Model 680 (Bio-Rad). A standard curve was prepared from the measurement results of the standard. The absorbance of each sample was fitted to a standard curve to obtain the concentration of IFNγ in each sample. The obtained value was defined as the IFNγ secretion amount of each effector cell and control cell.

### (2) Results

Table 2 shows the cytotoxic activity and IFNγ production when SK-MEL-37 is used as target cells and T cells including MAGE-A4-zG or mutants thereof are used as effector cells. Table 3 shows the cytotoxic activity and IFNγ production when SK-MEL-37 is used as target cells and T cells including MAGE-A4-zG-s1 or mutants thereof are used as effector cells. In the table, "type of CAR" is a code given to each CAR, and "WT" indicates an unmodified CAR (hereinafter, also referred to as "wild-type"). The code including "R" in "type of CAR" indicates a CAR in which the amino acid residue shown in "mutation site" in the table is substituted with an arginine residue. The "NA" in the column of "SEQ ID NO." indicates the SEQ ID NO. of the nucleotide sequence encoding the CAR, and the "AA" indicates the amino acid sequence of the CAR. The ratio when the ratio of the target cells killed by the CAR-T cells in which the type of CAR was WT was 1.00 was shown as the ratio of the number of effector cells to the number of target cells (hereinafter, the ratio was also referred to as "cell mixing ratio"), and the value of "IFNγ production" was shown as the ratio when the IFNγ production amount of the CAR-T cells in which the type of CAR was WT was 1.00. The same applies to the table of the following examples.

As shown in Table 2, the cytotoxic activity of each mutant when the cytotoxic activity of T cells including MAGE-A4-zG was 1.00 was as follows.
The cytotoxic activity of T cells including MAGE-A4-zG-m1 was 1.59 at a cell mixing ratio of 3 : 1 and 1.14 at a cell mixing ratio of 1 : 1.
The cytotoxic activity of T cells including MAGE-A4-zG-m2 is 1.41 at a cell mixing ratio of 3 : 1 and 1.60 at a cell mixing ratio of 1 : 1.
For cytotoxic activity, T cells with MAGE-A4-zG-m1 or MAGE-A4-zG-m2 were higher than T cells with MAGE-A4-zG.

On the other hand, when the IFNγ production amount of T cells including MAGE-A4-zG was 1.00, the IFNγ production amount of each mutant was as follows.
IFNγ production in T cells including MAGE-A4-zG-m1 was 0.67,
The IFNγ production of T cells including MAGE-A4-zG-m2 is 0.47.
For IFNγ production, T cells with MAGE-A4-zG-m1 or MAGE-A4-zG-m2 were lower than T cells with MAGE-A4-zG.

As shown in Table 3, the cytotoxic activity of T cells including MAGE-A4-zG-m1-s1 when the cytotoxic activity of T cells including MAGE-A4-zG-s1 was taken as 1.00 was 2.34 at a cell mixing ratio of 3 : 1 and 2.25 at a cell mixing ratio of 1 : 1. On the other hand, when the IFNγ production amount of the T cells including MAGE-A4-zG-s1 was 1.00, the IFNγ production amount of the T cells including MAGE-A4-zG-m1-s1 was 0.66. For cytotoxic activity, T cells with MAGE-A4-zG-m1-s1 were higher than T cells with MAGE-A4-zG-s1, but IFNγ production was lower than T cells with MAGE-A4-zG-s1.

**[Table 2]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | MAGE-A4-zG | 3 | 4 | NONE | 1.00 | 1.00 | 1.00 |
| R1234 | MAGE-A4-zG-m1 | 5 | 6 | 63, 65, 67, 70 | 1.59 | 1.14 | 0.67 |
| R1235 | MAGE-A4-zG-m2 | 7 | 8 | 63, 65, 67, 72 | 1.41 | 1.60 | 0.47 |

**[Table 3]**

| TYPE OF CAR | NAME OF CAR | SEQ ID NO | | MUTATION POSITION | CYTOTOXIC ACTIVITY | | IFN *γ* PRODUCTION |
|---|---|---|---|---|---|---|---|
| | | NA | AA | | 3:1 | 1:1 | |
| WT | MAGE-A4-zG-s1 | 9 | 10 | NONE | 1.00 | 1.00 | 1.00 |
| R1234 | MAGE-A4-zG-m1-s1 | 11 | 12 | 63, 65, 67, 70 | 2.34 | 2.25 | 0.66 |

Table 4 shows cytotoxic activity and IFNγ production when NW-MEL-38 is used as target cells and T cells including MAGE-A4-zG-s1 or MAGE-A4-zG-m1-s1 are used as effector cells. As shown in Table 4, the cytotoxic activity of T cells including MAGE-A4-zG-m1-s1 when the cytotoxic activity of T cells including MAGE-A4-zG-s1 was taken as 1.00 was 1.35 at a cell mixing ratio of 3 : 1 and 1.65 at a cell mixing ratio of 1: 1. For cytotoxic activity, T cells including MAGE-A4-zG-m1-s1 were higher than T cells including MAGE-A4-zG-s1. On the other hand, when the IFNγ production amount of the T cells including MAGE-A4-zG-s1 was 1.00, the IFNγ production amount of the T cells including MAGE-A4-zG-m1-s1 was 0.40. T cells including MAGE-A4-zG-m1-s1 were lower than T cells including MAGE-A4-zG-s1.

**[Table 4]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | MAGE-A4-zG-s1 | 9 | 10 | NONE | 1.00 | 1.00 | 1.00 |
| R1234 | MAGE-A4-zG-m1-s1 | 11 | 12 | 63, 65, 67, 70 | 1.35 | 1.65 | 0.40 |

It was suggested that the immune cell of the present example was a CAR-T cell in which cytokine production was suppressed. Further, the CAR-T cell had improved cytotoxicity.

### Example 2: Generation of CAR-T cells binding to MAGE-A4/fiLA-A2 complex and confirmation of their efficacy (2)

### (1) To obtain a nucleic acid molecule encoding a mutant of MAGE-A4-zG

A nucleic acid molecule encoding MAGE-A4-zG prepared in Example 1 was used as template DNA, and a nucleic acid molecule encoding a mutant different from that in Example 1 was produced by a PCR method. Specifically, it was as follows.

### (1.1) Primer design, PCR, and ligation reaction

A primer set for obtaining a polynucleotide encoding a CAR including a single-chain antibody in which the amino acid residues shown in a), c), e), f) or h) in MAGE-A4-zG below are substituted with arginine residues was designed based on the nucleotide sequence of SEQ ID NO: 3. A primer set for obtaining a polynucleotide encoding a CAR including a single-chain antibody in which the amino acid residues shown in b), d) or g) in MAGE-A4-zG were substituted with lysine residues was designed based on the nucleotide sequence of SEQ ID NO: 3. Using these primer sets and template DNA, PCR and a ligation reaction were performed in the same manner as in Example 1.

a) Amino acid residues at positions 63, 65 and 72 of the VL as defined by Kabat;
b) Amino acid residues at positions 63, 67 and 70 of the VL as defined by Kabat;
c) Amino acid residues at positions 63,70 and 72 of the VL as defined by Kabat;
d) Amino acid residues at positions 65, 67 and 70 of the VL as defined by Kabat;
e) Amino acid residues at positions 65, 70 and 72 of the VL as defined by Kabat;
f) Amino acid residues at positions 67, 70 and 72 of the VL as defined by Kabat;
g) Amino acid residues at positions 63,65,67,70 and 72 of the VL as defined by Kabat; and
h) Amino acid residues at positions 60, 74 and 76 of the VL as defined by Kabat

### (1.2) transformation, extraction of plasmid DNA and sequencing

In the same manner as in Example 1, a transformant of E. coli was obtained using the solution after the ligation reaction and DH5α. Then, in the same manner as in Example 1, plasmid DNA was extracted from E. coli cultured in a liquid medium, and sequencing was performed. Sequencing confirmed that a nucleic acid molecule encoding a mutant of MAGE-A4-zG was obtained.

Hereinafter, the CARs having a single-chain antibody in which the amino acid residues shown in a), c), e), f) or h) in the above MAGE-A4-zG are substituted with arginine residues are referred to as "MAGE-A4-zG-m5", "MAGE-A4-zG-m7", "MAGE-A4-zG-m9", "MAGE-A4-zG-m10" and "MAGE-A4-zG-m12", respectively. The CARs having a single-chain antibody in which the amino acid residues shown in b), d) or g) are substituted with lysine residues are referred to as "MAGE-A4-zG-Km6", "MAGE-A4-zG-Km8" and "MAGE-A4-zG-Km11", respectively.
Table 5 and Table 6 show the correspondence between each mutant and the SEQ ID NO. of the primer set for production thereof. In Table 6, "the first" indicates a primer set for substituting the amino acid residues at posistions 74 and 76 of the VL, and "the second" indicates a primer set for substituting the amino acid residue at position 60 of the VL.

**[Table 5]**

| NAME OF CAR | F | R |
|---|---|---|
| MAGE-A4-zG-m5 | 83 | 84 |
| MAGE-A4-zG-Km6 | 85 | 86 |
| MAGE-A4-zG-m7 | 87 | 88 |
| MAGE-A4-zG-Km8 | 89 | 90 |
| MAGE-A4-zG-m9 | 91 | 92 |
| MAGE-A4-zG-m10 | 93 | 94 |
| MAGE-A4-zG-Km11 | 95 | 96 |

**[Table 6]**

| NAME OF CAR | FIRST TIME | | SECOND TIME | |
|---|---|---|---|---|
| | F | R | F | R |
| MAGE-A4-zG-m12 | 97 | 98 | 99 | 100 |

### (2) Generation of CAR-T cells expressing MAGE-A4-zG and mutants thereof

In the same manner as in Example 1, a gene encoding MAGE-A4-zG and mutants thereof was introduced into PBMCs separated and cultured from blood of healthy donors using a retrovirus to obtain respective CAR-T cells.

### (3) Confirmation of cytotoxicity of CAR-T cells and cytokine secretion amount

The cytotoxic activity and IFNγ production of each CAR-T cell were measured in the same manner as in Example 1. SK-MEL-37 was used for the target cells. Measurements of MAGE-A4-zG-m5,MAGE-A4-zG-Km6,MAGE-A4-zG-m7,MAGE-A4-zG-Km8,MAGE-A4-zG-m9 and MAGE-A4-zG-m10 and measurements of MAGE-A4-zG-Km11 and MAGE-A4-zG-m12 were performed on different days. The results are shown in Tables 7 and 8. In the "type of CAR" in the table, the code including "K" indicates a CAR in which the amino acid residue shown in the "mutation site" in the table is substituted with a lysine residue. The same applies to the table of the following examples.

**[Table 7]**

| TYPE OF CAR | NAME OF CAR | SEQ ID NO | | MUTATION POSITION | CYTOTOXIC ACTIVITY | | IFN *γ* PRODUCTION |
|---|---|---|---|---|---|---|---|
| | | NA | AA | | 3:1 | 1:1 | |
| WT | MAGE-A4-zG | 3 | 4 | NONE | 1.00 | 1.00 | 1.00 |
| R125 | MAGE-A4-zG-m5 | 13 | 14 | 63, 65, 72 | 1.62 | 1.05 | 0.43 |
| K134 | MAGE-A4-zG-Km6 | 15 | 16 | 63, 67, 70 | 1.52 | 1.61 | 0.93 |
| R145 | MAGE-A4-zG-m7 | 17 | 18 | 63, 70, 72 | 1.77 | 0.99 | 0.22 |
| K234 | MAGE-A4-zG-Km8 | 19 | 20 | 65, 67, 70 | 1.47 | 1.36 | 0.91 |
| R245 | MAGE-A4-zG-m9 | 21 | 22 | 65, 70, 72 | 1.61 | 1.23 | 0.29 |
| R345 | MAGE-A4-zG--m10 | 23 | 24 | 67, 70, 72 | 1.77 | 0.99 | 0.32 |

**[Table 8]**

| TYPE OF CAR | NAME OF CAR | SEQ ID NO | | MUTATION POSITION | CYTOTOXIC ACTIVITY | | IFN *γ* PRODUCTION |
|---|---|---|---|---|---|---|---|
| | | NA | AA | | 3:1 | 1:1 | |
| WT | MAGE-A4-zG | 3 | 4 | NONE | 1.00 | 1.00 | 1.00 |
| K5 | MAGE-A4-zG-Km11 | 25 | 26 | 63, 65, 67, 70, 72 | 2.33 | 1.68 | 0.89 |
| R60_74_76 | MAGE-A4-zG-m12 | 27 | 28 | 60, 74, 76 | 1.23 | 0.90 | 0.40 |

As shown in Table 7, the cytotoxic activity of each mutant when the cytotoxic activity of T cells including MAGE-A4-zG was 1.00 was as follows.
The cytotoxic activity of T cells including MAGE-A4-zG-m5 was 1.62 at a cell mixing ratio of 3 : 1 and 1.05 at a cell mixing ratio of 1 : 1.
The cytotoxic activity of T cells including MAGE-A4-zG-Km6 was 1.52 at a cell mixing ratio of 3 : 1 and 1.61 at a cell mixing ratio of 1 : 1.
The cytotoxic activity of T cells including MAGE-A4-zG-m7 was 1.77 at a cell mixing ratio of 3 : 1 and 0.99 at a cell mixing ratio of 1 : 1.
The cytotoxic activity of T cells including MAGE-A4-zG-Km8 was 1.47 at a cell mixing ratio of 3 : 1 and 1.36 at a cell mixing ratio of 1 : 1.
The cytotoxic activity of T cells including MAGE-A4-zG-m9 was 1.61 at a cell mixing ratio of 3 : 1 and 1.23 at a cell mixing ratio of 1 : 1.
The cytotoxic activity of T cells including MAGE-A4-zG-m10 is 1.77 at a cell mixing ratio of 3 : 1 and 0.99 at a cell mixing ratio of 1 : 1.
On the other hand, when the IFNγ production amount of T cells including MAGE-A4-zG was 1.00, the IFNγ production amount of each mutant was as follows.
The IFNγ production of T cells including MAGE-A4-zG-m5 is 0.43,
The IFNγ production of T cells including MAGE-A4-zG-Km6 is 0.93,
The IFNγ production of T cells including MAGE-A4-zG-m7 is 0.22,
The IFNγ production of T cells including MAGE-A4-zG-Km8 is 0.91,
The IFNγ production of T cells including MAGE-A4-zG-m9 is 0.29,
The IFNγ production level of the T cells including MAGE-A4-zG-m10 is 0.32.

As shown in Table 8, the cytotoxic activity of each mutant when the cytotoxic activity of T cells including MAGE-A4-zG was 1.00 was as follows.
The cytotoxic activity of T cells including MAGE-A4-zG-Km11 was 2.33 at a cell mixing ratio of 3 : 1 and 1.68 at a cell mixing ratio of 1 : 1.
The cytotoxic activity of T cells including MAGE-A4-zG-m12 is 1.23 at a cell mixing ratio of 3 : 1 and 0.90 at a cell mixing ratio of 1 : 1.
On the other hand, when the IFNγ production amount of T cells including MAGE-A4-zG was 1.00, the IFNγ production amount of each mutant was as follows.
The IFNγ production of T cells including MAGE-A4-zG-Km11 is 0.89,
The IFNγ production level of the T cells including MAGE-A4-zG-m12 is 0.40.

It was suggested that the immune cell of the present example was a CAR-T cell in which cytokine production was suppressed. In addition, the cytotoxicity was improved in many clones. In MAGE-A4-zG-m12, cytotoxicity was slightly reduced as compared with that of the wild-type, but cytokine production was greatly suppressed. It was suggested that the clone could maintain the cytotoxicity more or to the same extent than wild-type by increasing the dose, and suppress cytokine production from wild-type.

### Example 3: Generation of CAR-T cells binding to PRAME/HLA-A24 complex and confirmation of their efficacy

### (1) Acquisition of nucleic acid molecule encoding CAR as template

In the same manner as in Example 1, plasmid DNA for producing a viral vector including a gene encoding a CAR having a single-chain antibody that binds to PRAME/fiLA-A24 complex was obtained. The complex recognized by the single-chain antibody was a complex of PRAME and HLA-A*24:02. Hereinafter, the CAR encoded by the gene in the plasmid DNA is referred to as "PRAME-zG". The amino acid sequence of the PRAME-derived peptide in the above PRAME/fiLA-A24 complex was LYVDSLFFL (SEQ ID NO: 2). In order to produce a nucleic acid molecule encoding a mutant of PRAME-zG by a PCR method, the above plasmid DNA was used as a template.

The configuration of the gene encoding PRAME-zG was the same as the gene encoding MAGE-A4-zG of Example 1. That is, in the gene encoding PRAME-zG, from the 5' end, a leader sequence, a nucleotide sequence encoding VH, a nucleotide sequence encoding a linker, a nucleotide sequence encoding VL, a nucleotide sequence encoding CL, a nucleotide sequence encoding CD28TM, a nucleotide sequence encoding CD3ζ, and a nucleotide sequence encoding GITRICD were linked in this order (see Fig. 2A). PRAME-zG was a CAR including, as extracellular domains, a single-chain antibody consisting of VH, VL and a linker connecting them, and CL, including CD28TM as a transmembrane domain, and including CD3ζ and GITRICD as an intracellular domain.

### (2) Obtainment of a nucleic acid molecule encoding a mutant of PRAME-zG

The above plasmid DNA was used as a template. A PCR reaction solution including template DNA, a primer set (SEQ ID NOs: 101 and 102) and a PrimeSTAR (registered trademark) Max Premix was prepared, and a PCR reaction was performed. Thus, a PCR product in which restriction sites were added to the 5' end and the 3' end was obtained. DpnI was added to the resulting PCR product to fragment the template plasmid DNA. A nucleic acid molecule encoding a mutant of PRAME-zG was produced by a PCR method using the DpnI-treated PCR product as a template. Specifically, it was as follows.

### (2.1) Primer design, PCR, and ligation reaction

A primer set for obtaining a polynucleotide encoding a CAR including a single-chain antibody in which the amino acid residues shown in the following a) or b) in PRAME-zG were substituted with arginine residues was designed based on the nucleotide sequence of SEQ ID NO: 29. A primer set for obtaining a polynucleotide encoding a CAR including a single-chain antibody in which the amino acid residue shown in the following c) in PRAME-zG was substituted with a lysine residue was designed based on the nucleotide sequence of SEQ ID NO: 29. Using these primer sets for producing mutants and the above DpnI-treated PCR products, PCR and ligation reactions were performed in the same manner as in Example 1.

a) Amino acid residues at positions 63, 65 and 72 of the VL as defined by Kabat;
b) Amino acid residues at positions 63,70 and 72 of the VL as defined by Kabat; and
c) Amino acid residues at positions 65,70 and 72 of the VL as defined by Kabat

### (2.2) transformation, extraction of plasmid DNA and sequencing

In the same manner as in Example 1, a transformant of E. coli was obtained using the solution after the ligation reaction and DH5α. Then, in the same manner as in Example 1, plasmid DNA was extracted from E. coli cultured in a liquid medium, and sequencing was performed. As a result of sequencing, it was confirmed that a nucleic acid molecule encoding a mutant of PRAME-zG was obtained.

Hereinafter, CARs having a single-chain antibody in which the amino acid residues shown in a) or b) in PRAME-zG are substituted with arginine residues are referred to as "PRAME-zG-m1" and "PRAME-zG-m2", respectively. A CAR having a single-chain antibody in which the amino acid residue shown in c) is substituted with a lysine residue is referred to as a "PRAME-zG-Km3". Table 9 shows the correspondence between each mutant and the SEQ ID NO. of the primer set for production thereof.

**[Table 9]**

| NAME OF CAR | F | R |
|---|---|---|
| PRAME-zG-m1 | 103 | 104 |
| PRAME-zG-m2 | 105 | 106 |
| PRAME-zG-Km3 | 107 | 108 |

### (3) Generation of CAR-T cells expressing PRAME-zG and mutants thereof

In the same manner as in Example 1, a gene encoding PRAME-zG and mutants thereof was introduced into PBMCs separated and cultured from blood of a healthy donor using a retrovirus to obtain each CAR-T cell.

### (4) Confirmation of cytotoxicity of CAR-T cells and cytokine secretion amount

The cytotoxic activity and IFNγ production of each CAR-T cell were measured in the same manner as in Example 1. For the target cells, a human melanoma cell line SK-MEL-124 was used. SK-MEL-124 was a PRAME positive tumor cell. Measurements of PRAME-zG-m1 and PRAME-zG-m2 were performed on a date different from measurement of PRAME-zG-Km3. The results are shown in Tables 10 and 11.

**[Table 10]**

| TYPE OF CAR | NAME OF CAR | SEO ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | PRAME-zG | 29 | 30 | NONE | 1.00 | 1.00 | 1.00 |
| R125 | PRAME-zG-m1 | 31 | 32 | 63, 65, 72 | 2.96 | 2.37 | 0.49 |
| R145 | PRAME-zG-m2 | 33 | 34 | 63, 70, 72 | 1.84 | 1.54 | 0.56 |

**[Table 11]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | PRAME-zG | 29 | 30 | NONE | 1.00 | 1.00 | 1.00 |
| K245 | PRAME-zG-Km3 | 35 | 36 | 65, 70, 72 | 1.16 | 1.71 | 0.87 |

As shown in Table 10, the cytotoxic activity of each mutant when the cytotoxic activity of T cells including PRAME-zG was taken as 1.00 was as follows.
The cytotoxic activity of T cells including PRAME-zG-m1 was 2.96 at a cell mixing ratio of 3 : 1 and 2.37 at a cell mixing ratio of 1 : 1.
The cytotoxic activity of T cells including PRAME-zG-m2 is 1.84 at a cell mixing ratio of 3 : 1 and 1.54 at a cell mixing ratio of 1 : 1.

On the other hand, the IFNγ production amount of each mutant when the IFNγ production amount of T cells including PRAME-zG was 1.00 was as follows.
The IFNγ production of T cells including PRAME-zG-m1 is 0.49,
The IFNγ production level of the T cells including PRAME-zG-m2 is 0.56.

As shown in Table 11, the cytotoxic activity of T cells including PRAME-zG-Km3 when the cytotoxic activity of T cells including PRAME-zG was taken as 1.00 was 1.16 at a cell mixing ratio of 3 : 1 and 1.71 at a cell mixing ratio of 1 : 1. On the other hand, when the IFNγ production amount of the T cells including PRAME-zG was 1.00, the IFNγ production amount of the T cells including PRAME-zG-Km3 was 0.87.

It was suggested that the immune cell of the present example was a CAR-T cell in which cytokine production was suppressed. Further, the CAR-T cell had improved cytotoxicity.

### Example 4: Generation of CAR-T cells binding to CD19 and confirmation of their effects

### (1) Acquisition of nucleic acid molecule encoding CAR as template

By gene synthesis, a polynucleotide (SEQ ID NO: 186) encoding a single-chain antibody that binds to CD19 (hereinafter, also referred to as "anti-CD19-WT") was obtained. As a mutant of anti-CD19-WT, polynucleotides (SEQ ID NOs: 187,188 and 190) encoding a single-chain antibody in which the amino acid residues shown in any of the following a) to c) in anti-CD19-WT were substituted with arginine residues were synthesized.
Hereinafter, the single-chain antibody in which the amino acid residues shown in the following a) to c) are modified is also referred to as "anti-CD19-m1", "anti-CD19-m2" and "anti-CD19-m4", respectively.

a) Amino acid residues at positions 63, 65, 67 and 70 of the VL as defined by Kabat;
b) Amino acid residues at positions 63, 65, 67 and 72 of the VL as defined by Kabat; and
c) Amino acid residues at positions 65, 67, 70 and 72 of the VL as defined by Kabat

### (2) Obtainment of a nucleic acid molecule encoding a mutant of CD19-28z

### (2.1) Production of the first insert and the second insert

Using a polynucleotide encoding each of the anti-CD19-WT, the anti-CD19-m1, the anti-CD19-m2, and the anti-CD19-m4 as a template, a PCR reaction solution including a primer set (SEQ ID NOs: 109 and 110) and a PrimeSTAR (registered trademark) Max Premix was prepared, and a PCR reaction was performed. This resulted in a first insert encoding each of anti-CD19-WT, anti-CD19-m1, anti-CD 19-m2 and anti-CD 19-m4. By gene synthesis, a polynucleotide encoding the hinge domain, the transmembrane domain, and the intracellular domain of CD28 (SEQ ID NO: 111) was obtained. Using this polynucleotide as a template, a PCR reaction solution including a primer set (SEQ ID NOs: 112 and 113) and a PrimeSTAR (registered trademark) Max Premix was prepared, and a PCR reaction was performed. Thus, a second insert encoding a region consisting of the hinge domain, the transmembrane domain, and the intracellular domain of CD28 was obtained.

### (2.2) Generation of linearized vector and fusion with insert

Using plasmid DNA (SEQ ID NO: 114) for producing a viral vector including a transmembrane domain of CD28 and a polynucleotide encoding an intracellular domain as a template, a PCR reaction solution including a primer set (SEQ ID NOs: 115 and 116) and a PrimeSTAR (registered trademark) Max Premix was prepared, and a PCR reaction was performed. DpnI was added to the resulting PCR product to fragment the template plasmid DNA. Thus, a linearized vector was obtained. This linearized vector and the first insert and the second insert were fused using an In-Fusion (registered trademark) HD Enzyme Premix (Takara Bio Inc.). Thus, plasmid DNA including a gene encoding a CAR including each of anti-CD19-WT, anti-CD19-m1, anti-CD19-m2, and anti-CD19-m4 was obtained. Hereinafter, CARs having single-chain antibodies of anti-CD19-WT, anti-CD19-m1, anti-CD19-m2, and anti-CD19-m4 are referred to as "CD19-28z", "CD19-28z-m1", "CD19-28z-m2", and "CD19-28z-m4", respectively.

As an example, the structure of a gene encoding CD19-28z is described. Referring to Fig. 2B, in the gene encoding CD19-28z, from the 5' end, a leader sequence, a nucleotide sequence encoding VL, a nucleotide sequence encoding a linker, a nucleotide sequence encoding VH, a nucleotide sequence encoding a hinge domain (CD28 hinge) of CD28, a nucleotide sequence encoding CD28TM, a nucleotide sequence encoding an intracellular domain (CD28ICD) of CD28, and a nucleotide sequence encoding CD3ζ were linked in this order. CD19-28z was a CAR including, as extracellular domains, a single-chain antibody consisting of VH, VL and a linker connecting them, and a hinge domain of CD28, including CD28TM as a transmembrane domain, and including CD28ICD and CD3ζ as intracellular domains.

### (2.3) Primer design, PCR, and ligation reaction

A primer set for obtaining a polynucleotide encoding a CAR including a single-chain antibody in which any of the following d), e), g), k) and q) in CD19-28z was substituted with an arginine residue was designed based on the nucleotide sequence of SEQ ID NO: 37. A primer set for obtaining a polynucleotide encoding a CAR including a single-chain antibody in which the amino acid residues shown in the following d), f), l) or m) in CD19-28z were substituted with lysine residues was designed based on the nucleotide sequence of SEQ ID NO: 37. Using these primer sets and template DNA, PCR and a ligation reaction were performed in the same manner as in Example 1.

d) Amino acid residues at positions 63, 65, 70 and 72 of the VL as defined by Kabat;
e) Amino acid residues at positions 60, 63 and 65 of the VL as defined by Kabat;
f) Amino acid residues at positions 63, 65 and 70 of the VL as defined by Kabat;
g) Amino acid residues at positions 63, 65 and 72 of the VL as defined by Kabat;
h) Amino acid residues at positions 63, 67 and 70 of the VL as defined by Kabat;
i) Amino acid residues at positions 63,70 and 72 of the VL as defined by Kabat;
j) Amino acid residues at positions 65, 67 and 70 of the VL as defined by Kabat;
k) Amino acid residues at positions 65, 67 and 72 of the VL as defined by Kabat;
l) Amino acid residues at positions 65, 67, 70 and 72 of the VL as defined by Kabat;
m) Amino acid residues at positions 63,65,67,70 and 72 of the VL as defined by Kabat;
n) Amino acid residues at positions 70, 72 and 74 of the VL as defined by Kabat;
o) Amino acid residues at positions 60, 63 and 76 of the VL as defined by Kabat;
p) Amino acid residues at positions 60, 74 and 76 of the VL as defined by Kabat; and
q) Amino acid residues at positions 77, 79 and 81 of the VL as defined by Kabat

### (2.2) transformation, extraction of plasmid DNA and sequencing

In the same manner as in Example 1, a transformant of E. coli was obtained using the solution after the ligation reaction and DH5α. Then, in the same manner as in Example 1, plasmid DNA was extracted from E. coli cultured in a liquid medium, and sequencing was performed. As a result of sequencing, it was confirmed that a nucleic acid molecule encoding a mutant of CD19-28z was obtained.

Hereinafter, the CARs having a single-chain antibody in which the amino acid residues shown in any of d), e), g) to k) to q) in CD19-28z are substituted with arginine residues are referred to as "CD19-28z-m3", "CD19-28z-m5", "CD19-28z-m7", "CD19-28z-m8", "CD19-28z-m10", "CD19-28z-m11", "CD19-28z-m12", "CD19-28z-m16", "CD19-28z-m17", "CD19-28z-m18", "CD19-28z-m19" and "CD19-28z-m20", respectively. The CARs having a single-chain antibody in which the amino acid residues shown in d), f), l) or m) are substituted with lysine residues are referred to as "CD19-28z-Km13", "CD19-28z-Km6", "CD19-28z-Km14" and "CD19-28z-Km15", respectively. Table 12 and Table 13 show the correspondence between each mutant and the SEQ ID NO. of the primer set for production thereof. Regarding CD19-28z-m18 in Table 13, "the first time" indicates a primer set for substituting the amino acid residues at positions 60 and 63 of the VL, and "the second time" indicates a primer set for substituting the amino acid residue at position 76 of the VL. With respect to CD19-28z-m19, "first time" indicates a primer set for substituting the amino acid residues at positions 74 and 76 of the VL, and "second time" indicates a primer set for substituting the amino acid residue at position 60 of the VL.

**[Table 12]**

| NAME OF CAR | F | R |
|---|---|---|
| CD19-28z-m3 | 117 | 118 |
| CD 19-28z-m5 | 119 | 120 |
| CD19-28z-Km6 | 121 | 122 |
| CD19-28z-m7 | 123 | 124 |
| CD 19-28z-m8 | 125 | 126 |
| CD19-28z-m10 | 127 | 128 |
| CD19-28z-m11 | 129 | 130 |
| CD19-28z-m12 | 131 | 132 |
| CD19-28z-Km13 | 133 | 134 |
| CD19-28z-Km14 | 135 | 136 |
| CD19-28z-Km15 | 137 | 138 |
| CD19-28z-m16 | 139 | 140 |
| CD19-28z-m17 | 141 | 142 |
| CD19-28z-m20 | 151 | 152 |

**[Table 13]**

| NAME OF CAR | FIRST TIME | | SECOND TIME | |
|---|---|---|---|---|
| | F | R | F | R |
| CD19-28z-m18 | 143 | 144 | 145 | 146 |
| CD19-28z-m19 | 147 | 148 | 149 | 150 |

### (3) Generation of CAR-T cells expressing CD19-28z and mutants thereof

In the same manner as in Example 1, a gene encoding CD19-28z and mutants thereof was introduced into PBMCs separated and cultured from blood of a healthy donor using a retrovirus to obtain each CAR-T cell.

### (4) Confirmation of cytotoxicity of CAR-T cells and cytokine secretion amount

The cytotoxic activity and IFNγ production of each CAR-T cell were measured in the same manner as in Example 1. In some experiments, the ratio of the number of cells of the effector cells to the target cells was 2 : 1 (see Table 16). For the target cells, a human B precursor cell leukemia cell line Nalm-6 was used. Nalm-6 was CD19 positive tumor cells. The results of the measurement are shown in Tables 14 to 17. The results of each table were based on measurements made on different days from each other.

**[Table 14]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | CD19-28z | 37 | 38 | NONE | 1.00 | 1.00 | 1.00 |
| R1234 | CD19-28z-m1 | 39 | 40 | 63, 65, 67, 70 | 1.03 | 1.30 | 0.25 |
| R1235 | CD19-28z-m2 | 41 | 42 | 63, 65, 67, 72 | 0.90 | 1.19 | 0.24 |
| R1245 | CD19-28z-m3 | 43 | 44 | 63, 65, 70, 72 | 0.91 | 1.26 | 0.26 |

**[Table 15]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | CD19-28z | 37 | 38 | NONE | 1.00 | 1.00 | 1.00 |
| R1234 | CD19-28z-m1 | 39 | 40 | 63, 65, 67, 70 | 1.00 | 1.56 | 0.31 |
| R1245 | CD19-28z-m3 | 43 | 44 | 63, 65, 70, 72 | 1.09 | 1.37 | 0.27 |
| R2345 | CD19-28z-m4 | 45 | 46 | 65, 67, 70, 72 | 1.17 | 1.49 | 0.29 |

**[Table 16]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 2:1 | 1:1 | |
| WT | CD19-28z | 37 | 38 | NONE | 1.00 | 1.00 | 1.00 |
| R60_63_65 | CD19-28z-m5 | 47 | 48 | 60, 63, 65 | 1.09 | 0.95 | 0.34 |
| K124 | CD19-28z-Km6 | 49 | 50 | 63, 65, 70 | 1.50 | 1.13 | 0.20 |
| R125 | CD19-28z-m7 | 51 | 52 | 63, 65, 72 | 1.58 | 1.11 | 0.13 |
| R134 | CD19-28z-m8 | 53 | 54 | 63, 67, 70 | 1.61 | 1.19 | 0.08 |
| R145 | CD19-28z-m10 | 55 | 56 | 63, 70, 72 | 1.59 | 0.98 | 0.21 |
| R234 | CD19-28z-m11 | 57 | 58 | 65, 67, 70 | 1.40 | 1.06 | 0.17 |
| R235 | CD19-28z-m12 | 59 | 60 | 65, 67, 72 | 1.47 | 1.39 | 0.16 |
| K1245 | CD19-28z-Km13 | 61 | 62 | 63, 65, 70, 72 | 1.68 | 1.37 | 0.13 |
| K2345 | CD19-28z-Km14 | 63 | 64 | 65, 67, 70, 72 | 1.72 | 1.34 | 0.13 |
| K5 | CD18-282z-Km15 | 65 | 66 | 63, 65, 67, 70, 72 | 1.29 | 1.23 | 0.14 |
| R5 | CD19-28z-m16 | 67 | 68 | 63, 65, 67, 70, 72 | 1.93 | 1.32 | 0.15 |

**[Table 17]**

| TYPE OF CAR | NAME OF CAR | SEQ ID NO | | MUTATION POSITION | CYTOTOXIC ACTIVITY | | IFN*γ* PRODUCTION |
|---|---|---|---|---|---|---|---|
| | | NA | AA | | 3:1 | 1:1 | |
| WT | CD19-28z | 37 | 38 | NONE | 1.00 | 1.00 | 1.00 |
| R70_72_74 | CD19-28z-m17 | 69 | 70 | 70, 72, 74 | 1.43 | 2.43 | 0.34 |
| R60_63_76 | CD19-28z-m18 | 71 | 72 | 60, 63, 76 | 1.27 | 1.42 | 0.28 |
| R60_74_76 | CD19-28z-m19 | 73 | 74 | 60, 74, 76 | 1.38 | 1.76 | 0.68 |
| R77_79_81 | CD19-28z-m20 | 75 | 76 | 77, 79, 81 | 1.57 | 1.90 | 0.39 |

As shown in Table 14, the cytotoxic activity of each mutant when the cytotoxic activity of T cells including CD19-28z was taken as 1.00 was as follows.
The cytotoxic activity of T cells including CD19-28z-m1 was 1.03 at a cell mixing ratio of 3 : 1 and 1.30 at a cell mixing ratio of 1 : 1.
The cytotoxic activity of T cells including CD19-28z-m2 was 0.90 at a cell mixing ratio of 3 : 1 and 1.19 at a cell mixing ratio of 1 : 1.
The cytotoxic activity of T cells including CD19-28z-m3 is 0.91 at a cell mixing ratio of 3 : 1 and 1.26 at a cell mixing ratio of 1 : 1.

On the other hand, the IFNγ production amount of each mutant when the IFNγ production amount of T cells including CD19-28z was taken as 1.00 was as follows.
The IFNγ production of T cells including CD19-28z-m1 is 0.25,
The IFNγ production of T cells including CD19-28z-m2 is 0.24,
The IFNγ production level of the T cells including CD19-28z-m3 is 0.26.

As shown in Table 15, the cytotoxic activity of each mutant when the cytotoxic activity of T cells including CD19-28z was taken as 1.00 was as follows.
The cytotoxic activity of T cells including CD19-28z-m1 was 1.00 at a cell mixing ratio of 3 : 1 and 1.56 at a cell mixing ratio of 1 : 1.
The cytotoxic activity of T cells including CD19-28z-m3 was 1.09 at a cell mixing ratio of 3 : 1 and 1.37 at a cell mixing ratio of 1 : 1.
The cytotoxic activity of T cells including CD19-28z-m4 is 1.17 at a cell mixing ratio of 3 : 1 and 1.49 at a cell mixing ratio of 1 : 1.

On the other hand, the IFNγ production amount of each mutant when the IFNγ production amount of T cells including CD19-28z was taken as 1.00 was as follows.
The IFNγ production of T cells including CD19-28z-m1 is 0.31,
The IFNγ production of T cells including CD19-28z-m3 is 0.27,
The IFNγ production level of the T cells including CD19-28z-m4 is 0.29.

As shown in Table 16, the cytotoxic activity of each mutant when the cytotoxic activity of T cells including CD19-28z was taken as 1.00 was as follows.
The cytotoxic activity of T cells including CD19-28z-m5 was 1.09 at a cell mixing ratio of 2 : 1 and 0.95 at a cell mixing ratio of 1 : 1.
The cytotoxic activity of T cells including CD19-28z-Km6 was 1.50 at a cell mixing ratio of 2 : 1 and 1.13 at a cell mixing ratio of 1 : 1.
The cytotoxic activity of T cells including CD19-28z-m7 was 1.58 at a cell mixing ratio of 2 : 1 and 1.11 at a cell mixing ratio of 1 : 1.
The cytotoxic activity of T cells including CD19-28z-m8 was 1.61 at a cell mixing ratio of 2 : 1 and 1.19 at a cell mixing ratio of 1 : 1.
The cytotoxic activity of T cells including CD19-28z-m10 was 1.59 at a cell mixing ratio of 2 : 1 and 0.98 at a cell mixing ratio of 1 : 1.
The cytotoxic activity of T cells including CD19-28z-m 11 was 1.40 at a cell mixing ratio of 2 : 1 and 1.06 at a cell mixing ratio of 1 : 1.
The cytotoxic activity of T cells including CD19-28z-m12 was 1.47 at a cell mixing ratio of 2 : 1 and 1.39 at a cell mixing ratio of 1 : 1.
The cytotoxic activity of T cells including CD19-28z-Km13 was 1.68 at a cell mixing ratio of 2 : 1 and 1.37 at a cell mixing ratio of 1 : 1.
The cytotoxic activity of T cells including CD19-28z-Km14 was 1.72 at a cell mixing ratio of 2 : 1 and 1.34 at a cell mixing ratio of 1 : 1.
The cytotoxic activity of T cells including CD19-28z-Km15 was 1.29 at a cell mixing ratio of 2 : 1 and 1.23 at a cell mixing ratio of 1 : 1.
The cytotoxic activity of T cells including CD19-28z-m16 is 1.93 at a cell mixing ratio of 2 : 1 and 1.32 at a cell mixing ratio of 1 : 1.

On the other hand, the IFNγ production amount of each mutant when the IFNγ production amount of T cells including CD19-28z was taken as 1.00 was as follows.
The IFNγ production of T cells including CD19-28z-m5 is 0.34,
The IFNγ production of T cells including CD19-28z-m6 is 0.20,
The IFNγ production of T cells including CD19-28z-m7 is 0.13,
The IFNγ production of T cells including CD19-28z-m8 is 0.08,
The IFNγ production of T cells including CD19-28z-m10 is 0.21,
The IFNγ production of T cells including CD19-28z-m11 is 0.17,
The IFNγ production of T cells including CD19-28z-m12 is 0.16,
The IFNγ production of T cells including CD19-28z-Km13 is 0.13,
The IFNγ production of T cells including CD19-28z-Km14 is 0.13,
The IFNγ production of T cells including CD19-28z-Km15 is 0.14,
The IFNγ production level of the T cells including CD19-28z-m16 is 0.15.

As shown in Table 17, the cytotoxic activity of each mutant when the cytotoxic activity of T cells including CD19-28z was taken as 1.00 was as follows.
The cytotoxic activity of T cells including CD19-28z-m17 was 1.43 at a cell mixing ratio of 3 : 1 and 2.43 at a cell mixing ratio of 1 : 1.
The cytotoxic activity of T cells including CD19-28z-m18 was 1.27 at a cell mixing ratio of 3 : 1 and 1.42 at a cell mixing ratio of 1 : 1.
The cytotoxic activity of T cells including CD19-28z-m19 was 1.38 at a cell mixing ratio of 3 : 1 and 1.76 at a cell mixing ratio of 1 : 1.
The cytotoxic activity of T cells including CD19-28z-m20 is 1.57 at a cell mixing ratio of 3 : 1 and 1.90 at a cell mixing ratio of 1 : 1.

On the other hand, the IFNγ production amount of each mutant when the IFNγ production amount of T cells including CD19-28z was taken as 1.00 was as follows.
The IFNγ production of T cells including CD19-28z-m17 is 0.34,
The IFNγ production of T cells including CD19-28z-m18 is 0.28,
The IFNγ production of T cells including CD19-28z-m19 is 0.68,
The IFNγ production level of the T cells including CD19-28z-m20 is 0.39.

As shown in Examples 1 to 4, the IFNγ production amount of the immune cell of the present invention showed a value of 0.08 to 0.93 when the wild-type was 1.00, and was lower than that of the wild-type. The cytotoxic activity of the CAR-T cells showed a value of 0.90 to 2.96 when the wild-type was taken as 1.00, and the cytotoxic activity was maintained or improved in many clones as compared with the wild-type.

### Example 5: In vivo cancer treatment model experiment by administration of CAR-T cells

### (1) Material and method

### (1.1) Experimental animal

NOG mice (NOD/Shi-scid,IL-2R yKOJic) were purchased from Nippon Clare Co.,Ltd. In the experiment, female mice of 7 to 8 weeks of age were used.

### (1.2) Transplantation of human tumors and administration of CAR-T cells

NW-MEL-38 was used as a tumor cell. As effector cells, CAR-T cells including MAGE-A4-zG-s1 prepared in Example 1 and CAR-T cells including MAGE-A4-zG-m1-s1 were used. Twelve NOG mice were injected subcutaneously with NW-MEL-38 in 5×10 ⁶ cells/animal. Four days after transplantation of tumor cells, T cells including PBS,MAGE-A4-zG-s1 or CAR-T cells including MAGE-A4-zG-m1-s1 were infused from the tail vein with 5×10 ⁶ cells/animal. The group administered with PBS, the group administered with CAR-T cells including MAGE-A4-zG-s1, and the group administered with CAR-T cells including MAGE-A4-zG-m1-s1 were all n = 4. The tumor diameter was measured every 2 days or 3 days.

### (2) Results

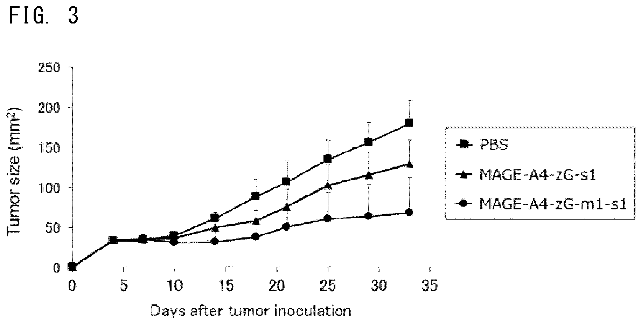

A graph in which the average value of the tumor area of each group is recorded is shown in Fig. 3. As can be seen from Fig. 3, as compared with the group administered with PBS, the tumor diameter was reduced in the group administered with CAR-T cells including MAGE-A4-zG-s1 and the group administered with CAR-T cells including MAGE-A4-zG-m1-s1. In the group administered with the CAR-T cells including MAGE-A4-zG-m1-s1, the tumor-diameter was smaller than that in the group administered with the CAR-T cells including MAGE-A4-zG-s1. Therefore, it was suggested that the antitumor activity of the immune cell including the CAR can be improved by changing at least three amino acid residues of the light chain FR3 of the single-chain antibody included in the CAR to basic amino acid residues.

## Claims

1. A nucleic acid molecule comprising a nucleotide sequence encoding a chimeric antigen receptor, wherein
the nucleic acid molecule comprises a segment encoding an extracellular domain, a segment encoding a transmembrane domain, and a segment encoding an intracellular domain,
the segment encoding the extracellular domain comprises a nucleotide sequence encoding an antigen binding region comprising a light chain variable region and a heavy chain variable region, and
at least three codons in the nucleotide sequence encoding framework region 3 of the light chain variable region defined by the Kabat method are codons encoding basic amino acid residues.

2. The nucleic acid molecule according to claim 1, wherein said at least three codons comprise at least three codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, a codon encoding an amino acid residue at position 76, a codon encoding an amino acid residue at position 77, a codon encoding an amino acid residue at position 79, and a codon encoding an amino acid residue at position 81 of the light chain variable region.

3. The nucleic acid molecule according to claim 1, wherein 3 or more and 5 or less codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, a codon encoding an amino acid residue at position 76, a codon encoding an amino acid residue at position 77, a codon encoding an amino acid residue at position 79, and a codon encoding an amino acid residue at position 81 of the light chain variable region amino acid residue at position 60amino acid residue at position 63amino acid residue at position 65amino acid residue at position 67amino acid residue at position 70tamino acid residue at position 72amino acid residue at position 74amino acid residue at position 76amino acid residue at position 77amino acid residue at position 79amino acid residue at position 81 defined by the Kabat method are codons encoding basic amino acid residues.

4. The nucleic acid molecule according to claim 1, wherein the antigen binding region comprises a single-chain antibody, and the single-chain antibody is a single-chain antibody that binds to: a complex of a MAGE-A4 derived peptide and HLA-A2, a complex of a PRAME derived peptide and HLA-A24, CD19, BCMA, or CEA.

5. The nucleic acid molecule according to claim 1, wherein the transmembrane domain comprises a transmembrane region of a protein selected from the group consisting of an α chain of a T cell receptor, a 3 chain of a T cell receptor, CD3ε, CD3ζ, CD4, CD5, CD8, CD9, CD16, CD22, CD28, CD33, CD37, CD45, CD64, CD80, CD86, CD134, CD154, 4-1BB, ICOS, and GITR.

6. The nucleic acid molecule according to claim 1, wherein the intracellular domain comprises a signaling domain of at least one protein selected from the group consisting of CD3ζ, CD3γ, CD3δ, CD3ε, CD5, CD22, CD66d, CD79a, CD79b, FcRγ, and FcRβ.

7. The nucleic acid molecule according to claim 6, wherein the segment encoding the intracellular domain further comprises a nucleotide sequence encoding a co-stimulatory domain, and
the co-stimulatory domain is a co-stimulatory domain of at least one protein selected from the group consisting of 4-1B B,CD28,GITR,CD2,CD5,CD8,CD9,CD16,CD22,CD33,CD37,CD64,CD80,CD86,CD 134, CD154, and ICOS.

8. The nucleic acid molecule according to claim 1, further comprising a segment encoding a hinge domain between the nucleotide sequence encoding the antigen binding region and the segment encoding the transmembrane domain.

9. The nucleic acid molecule according to claim 1, wherein the nucleic acid molecule is DNA or RNA.

10. A vector comprising the nucleic acid molecule according to any one of claims 1 to 9.

11. A chimeric antigen receptor comprising an extracellular domain, a transmembrane domain, and an intracellular domain, wherein
the extracellular domain comprises an antigen binding region comprising a light chain variable region and a heavy chain variable region, and
at least three amino acid residues in framework region 3 of the light chain variable region defined by the Kabat method are basic amino acid residues.

12. The chimeric antigen receptor according to claim 11, wherein said at least three amino acid residues comprise at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, an amino acid residue at position 76, an amino acid residue at position 77, an amino acid residue at position 79, and an amino acid residue at position 81 in the light chain variable region.

13. The chimeric antigen receptor according to claim 11, wherein 3 or more and 5 or less amino acid residues selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, an amino acid residue at position 76, an amino acid residue at position 77, an amino acid residue at position 79, and an amino acid residue at position 81 in the light chain variable region defined by the Kabat method are basic amino acid residues.

14. The chimeric antigen receptor according to claim 11, wherein the antigen binding region comprises a single-chain antibody, and the single-chain antibody is a single-chain antibody that binds to a complex of a MAGE-A4 derived peptide and HLA-A2, a complex of a PRAME derived peptide and HLA-A24, CD19, BCMA, or CEA.

15. The chimeric antigen receptor according to claim 11, wherein the transmembrane domain comprises a transmembrane region of any one protein selected from the group consisting of an α chain of a T cell receptor, a β chain of a T cell receptor, CD3ε, CD3ζ, CD4,CD5,CD8,CD9,CD16,CD22,CD28,CD33,CD37,CD45,CD64,CD80,CD86,CD 134, CD154,4-1BB,ICOS, and GITR.

16. The chimeric antigen receptor according to claim 11, wherein the intracellular domain comprises a signaling domain of at least one protein selected from the group consisting of CD3ζ, CD3γ, CD3δ, CD3ε, CD5,CD22,CD66d,CD79a,CD79b,FcRγ, and FcRβ.

17. The chimeric antigen receptor according to claim 16, wherein the intracellular domain further comprises a co-stimulatory domain, and
the co-stimulatory domain is a co-stimulatory domain of at least one protein selected from the group consisting of 4-1BB, CD28,GITR,CD2,CD5,CD8,CD9,CD16,CD22,CD33,CD37,CD64,CD80,CD86,CD 134, CD154, and ICOS.

18. The chimeric antigen receptor according to claim 11, further comprising a hinge domain between the antigen binding region and the transmembrane domain.

19. An immune cell comprising the chimeric antigen receptor according to any one of claims 11 to 18.

20. A pharmaceutical composition for treating a malignant tumor comprising the immune cell according to claim 19.

21. A method for producing an immune cell comprising a chimeric antigen receptor, comprising introducing the nucleic acid molecule according to any one of claims 1 to 9 into an immune cell, and expressing the chimeric antigen receptor in the immune cell.

22. A method for producing an immune cell comprising a chimeric antigen receptor, comprising introducing the vector according to claims 10 into an immune cell, and expressing the chimeric antigen receptor in the immune cell.
